(19) Europäisches Patentamt
European Patent Office
Office européen des brevets

(11) **EP 2 933 795 A1**

(12) **EUROPEAN PATENT APPLICATION**

(43) Date of publication:
**21.10.2015 Bulletin 2015/43**

(51) Int Cl.:
**G10K 11/34** *(2006.01)*     *A61B 8/00* *(2006.01)*

(21) Application number: **15162359.2**

(22) Date of filing: **02.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **14.04.2014 KR 20140044453**

(71) Applicant: **Samsung Electronics Co., Ltd.
Suwon-si, Gyeonggi-do, 443-742 (KR)**

(72) Inventors:
• **Park, Sungchan
  Gyeonggi-do (KR)**

• **Kang, Jooyoung
  Gyeonggi-do (KR)**
• **Kim, Kyuhong
  Seoul (KR)**
• **Kim, Jungho
  Gyeonggi-do (KR)**
• **Park, Suhyun
  Gyeonggi-do (KR)**

(74) Representative: **Grünecker Patent- und Rechtsanwälte
PartG mbB
Leopoldstraße 4
80802 München (DE)**

(54) **ULTRASONIC PROBE, ULTRASONIC IMAGING APPARATUS, AND METHOD OF CONTROLLING THE ULTRASONIC IMAGING APPARATUS**

(57)     An ultrasonic probe (100) includes an ultrasonic array (110) including ultrasonic elements configured to receive ultrasonic waves that are generated from target regions (t1 to t5) according to interference between the ultrasonic waves of different frequencies which are transmitted to same target regions; and a support frame on which the ultrasonic elements are arranged.

**FIG. 1**

**Description**

BACKGROUND

**1. Field**

Apparatuses and methods consistent with exemplary embodiments relate to an ultrasonic probe, an ultrasonic imaging apparatus, and a method of controlling the ultrasonic imaging apparatus.

**2. Description of the Related Art**

An ultrasonic imaging apparatus acquires internal images of an object such as a human body by transmitting ultrasonic waves to a target region inside the object, collecting ultrasonic waves (that is, echo ultrasonic waves) reflected from the target region, and generating an ultrasonic image based on the echo ultrasonic waves.

The ultrasonic imaging apparatus collects ultrasonic waves generated or reflected from the internal regions of an object through an ultrasonic probe, converts the ultrasonic waves into electrical signals, and generates an ultrasonic image corresponding to the collected ultrasonic waves based on the electrical signals. More specifically, the ultrasonic imaging apparatus may perform beamforming on the electrical signals to obtain ultrasonic signals, and generate an ultrasonic image based on the beamformed ultrasonic signals. The ultrasonic imaging apparatus may perform predetermined image processing on the ultrasonic image to generate an ultrasonic image of the object. The generated ultrasonic image may be displayed for a user, such as a doctor or a patient, through a display device such as a monitor installed in the ultrasonic imaging apparatus or connected to the ultrasonic imaging apparatus through a wired and/or wireless communication network.

SUMMARY

One or more exemplary embodiments provide an ultrasonic probe, an ultrasonic imaging apparatus, and a control method of the ultrasonic imaging apparatus, for quickly acquiring ultrasonic images.

One or more exemplary embodiments provide an ultrasonic probe, an ultrasonic imaging apparatus, and a control method of the ultrasonic imaging apparatus, for acquiring ultrasonic waves generated from the internal region of an object using ultrasound receiving elements, instead of a hydrophone, to generate an ultrasonic image.

In accordance with an aspect of an exemplary embodiment, an ultrasonic probe includes: an ultrasonic array including a plurality of ultrasonic elements configured to receive ultrasonic waves of low frequencies ranging from several Hz to several hundreds of KHz, generated by a radiation force from a plurality of target regions, according to interference between a plurality of ultrasonic waves of different frequencies ranging from several MHz to several tens of MHz transmitted to the same target regions; and a support frame on which the plurality of ultrasonic elements are arranged. A plurality of ultrasonic generating elements may transmit a plurality of ultrasonic waves of different frequencies to a plurality of target regions spaced by a predetermined distance, at the same time, or the plurality of ultrasonic generating elements may transmit the plurality of ultrasonic waves of the different frequencies to a plurality of different target regions, at the same time, several times.

The ultrasonic probe may further include an ultrasonic signal obtainer configured to acquire a plurality of ultrasonic signals for the plurality of target regions, based on the plurality of ultrasonic waves of the different frequencies and the received ultrasonic waves of the low frequencies ranging from several Hz to several hundreds of KHz.

In accordance with an aspect of an exemplary embodiment, an ultrasonic imaging apparatus includes: an ultrasonic probe including a plurality of ultrasonic elements configured to receive a plurality of ultrasonic waves that are generated from a plurality of target regions according to interference between a plurality of ultrasonic waves of different frequencies transmitted to the same target regions; and an ultrasonic signal obtainer configured to acquire a plurality of ultrasonic signals for the plurality of target regions, based on the plurality of ultrasonic waves of the different frequencies transmitted to the plurality of target regions and the received ultrasonic waves.

In accordance with an aspect of an exemplary embodiment, a control method of an ultrasonic imaging apparatus includes: transmitting a plurality of ultrasonic waves of different frequencies to a plurality of target regions, at the same time; at a plurality of ultrasonic elements, receiving a plurality of ultrasonic waves that are generated from the plurality of target regions according to interference between the plurality of ultrasonic waves of the different frequencies transmitted to the same target regions; acquiring a plurality of ultrasonic signals for the plurality of target regions based on the plurality of ultrasonic waves of the different frequencies and the generated ultrasonic waves; and generating an ultrasonic image based on the acquired ultrasonic signals, wherein the plurality of target regions may be spaced by a predetermined distance.

The control method of the ultrasonic imaging apparatus may generate a frequency that is equal to or higher than a frequency of second harmonic components at the plurality of target regions, or may use a frequency of harmonic components that are generated upon transmission to the ultrasonic probe. The control method may measure movement using the Doppler effect after transmitting pulse echo.

BRIEF DESCRIPTION OF THE DRAWINGS

The above and/or other aspects will become more apparent by describing certain exemplary embodiments, with reference to the accompanying drawings, in which:

FIG. 1 illustrates an ultrasonic probe according to an exemplary embodiment;
FIG. 2 is a block diagram illustrating a configuration of an ultrasonic probe according to an exemplary embodiment;
FIGS. 3A and 3B illustrate a configuration of an ultrasonic array according to an exemplary embodiment;
FIG. 4 is a perspective view illustrating an ultrasonic array according to an exemplary embodiment;
FIG. 5 is a view for describing interference between ultrasonic waves of different frequencies;
FIG. 6 is a view for describing the beating;
FIGS. 7A, 7B, and 7C are views for describing acquisition of ultrasonic signals according to an exemplary embodiment;
FIGS. 8A, 8B, and 8C are views for describing acquisition of ultrasonic signals according to an exemplary embodiment;
FIGS. 9 is a view for describing acquisition of ultrasonic signals according to an exemplary embodiment;
FIGS. 10A, 10B, and 10C are views for describing acquisition of ultrasonic signals according to an exemplary embodiment;
FIGS. 11 and 12 are views for describing acquisition of ultrasonic signals according to an exemplary embodiment;
FIG. 13 is a perspective view of an ultrasonic imaging apparatus according to an exemplary embodiment;
FIG. 14 is a block diagram illustrating a configuration of an ultrasonic imaging apparatus according to an exemplary embodiment;
FIG. 15 is a block diagram illustrating a beamformer according to an exemplary embodiment;
FIG. 16 is a block diagram illustrating a beamformer according to an exemplary embodiment;
FIG. 17 is a flowchart illustrating a control method of an ultrasonic imaging apparatus, according to an exemplary embodiment;
FIG. 18 is a flowchart illustrating a control method of an ultrasonic imaging apparatus, according to an exemplary embodiment;
FIG. 19 is a flowchart illustrating a control method of an ultrasonic imaging apparatus, according to an exemplary embodiment.

DETAILED DESCRIPTION

Certain exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

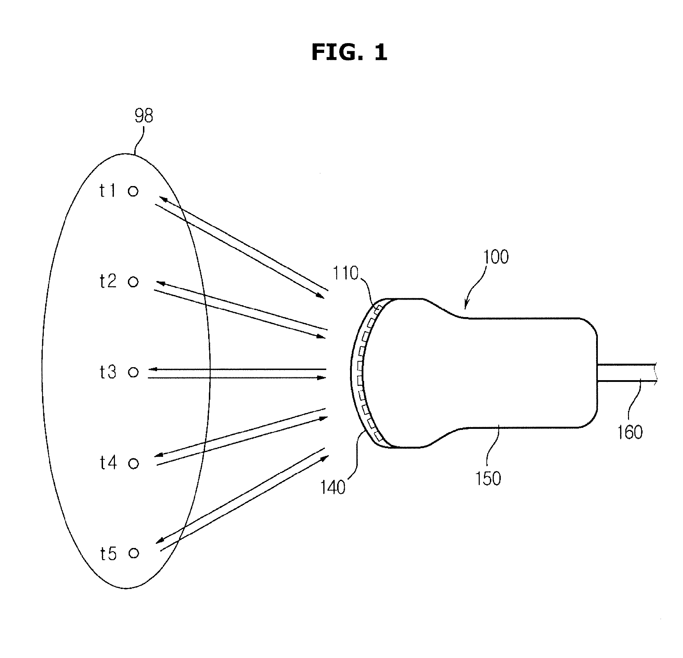

Hereinafter, an ultrasonic probe according to an exemplary embodiment will be described with reference to FIGS. 1 to 12.

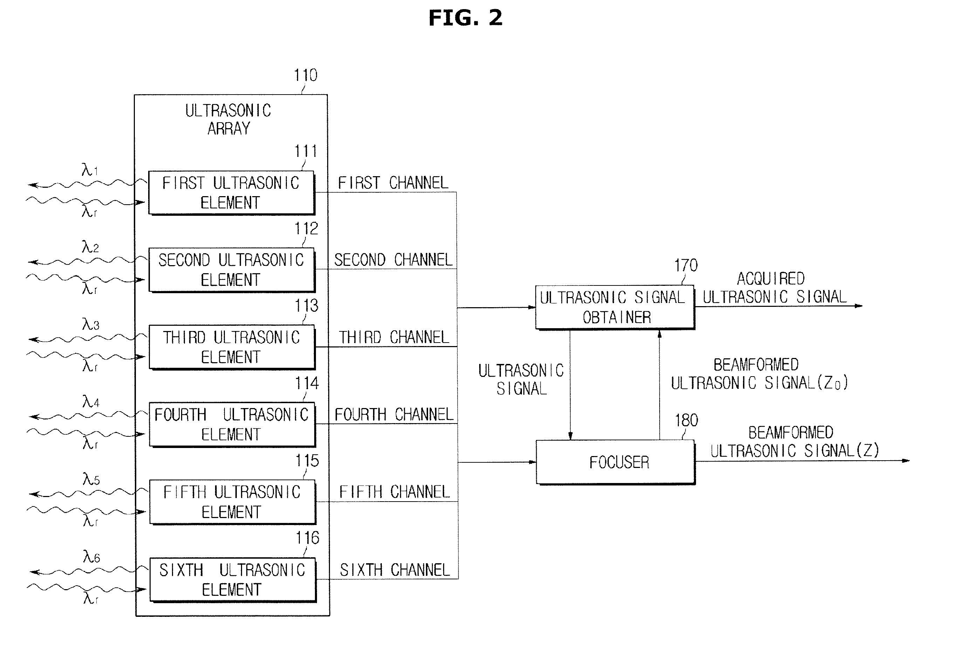

FIG. 1 illustrates an ultrasonic probe according to an exemplary embodiment, and FIG. 2 is a block diagram illustrating a configuration of an ultrasonic probe according to an exemplary embodiment. Referring to FIGS. 1 and 2, an ultrasonic probe 100 may receive waves, such as sound waves or ultrasonic waves, from a plurality of target regions t1 to t5 inside an object 98 to collect information about the internal regions of the object 98. In order to receive the waves, the ultrasonic probe 100 may include an ultrasonic array 110 to receive vibration waves from the outside, at an end of the ultrasonic probe 100, as shown in FIG. 1.

The ultrasonic array 110 may include, as shown in FIG. 2, a plurality of ultrasonic elements, for example, first to sixth ultrasonic elements 111 to 116. Each of the ultrasonic elements 111 to 116 may receive ultrasonic waves from the outside, and convert the ultrasonic waves into electrical signals, that is, ultrasonic signals. For example, as shown in FIG. 2, when ultrasonic waves of a predetermined frequency $\lambda_r$ are incident to the individual ultrasonic elements 111 to 116, piezoelectric vibrators (a piezoelectric material) or thin films of the individual ultrasonic elements 111 to 116 may vibrate at a frequency corresponding to the frequency $\lambda_r$ of the incident ultrasonic waves. When the piezoelectric vibrators or the thin films vibrate, the individual ultrasonic elements 111 to 116 may generate and output alternating current of a frequency corresponding to the vibration frequency of the vibrating piezoelectric vibrators or the thin films, thereby converting the incident ultrasonic waves into electrical signals. In this way, the ultrasonic array 110 may convert incident ultrasonic waves into the corresponding electrical signals.

The electrical signals output from the individual ultrasonic elements 111 to 116 of the ultrasonic array 110 may be transferred to an ultrasonic signal obtainer 170 or a focuser 180 through a plurality of channels, for example, first, second, third, fourth, fifth and sixth channels, as shown in FIG. 2.

According to an exemplary embodiment, the ultrasonic array 110 may receive ultrasonic waves according to a radiation force, e.g., an acoustic radiation force, of the internal tissue of the object 98. For example, the ultrasonic array 110 may receive ultrasonic waves of low frequencies ranging from several Hz to several hundreds of kHz.

According to an exemplary embodiment, the ultrasonic elements 111 to 116 may generate ultrasonic waves of predetermined frequencies. More specifically, if pulse current of a predetermined frequency is applied to the individual ultrasonic elements 111 to 116, as shown in FIGS. 1 and 2, the ultrasonic elements 111 to 116 may vibrate at a frequency corresponding to the frequency of the applied pulse current, and generate ultrasonic waves of predetermined frequencies $\lambda_1$ to $\lambda_6$ according to the vibration. For example, all the frequencies of the ultrasonic waves, for example, the first to sixth frequencies $\lambda_1$ to $\lambda_6$ generated by the individual ultrasonic elements 111 to 116 may be not the same. That is, a part of the frequencies $\lambda_1$ to $\lambda_6$ may be different from the other part of the frequencies $\lambda_1$ to $\lambda_6$. According to an exemplary embodiment, each of the ultrasound frequencies $\lambda_1$ to $\lambda_6$ generated by the individual ultrasonic elements, for example, the first to sixth ultrasonic elements 111 to 116 may be different from each other.

The individual ultrasonic elements 111 to 116 may transmit the ultrasonic waves to at least one target region at the same time. Herein, transmitting ultrasonic waves at the same time may include transmitting ultrasonic waves at the exactly same time and transmitting ultrasonic waves with a small time difference. For example, some of the ultrasonic elements 111 to 116 may transmit ultrasonic waves at the same time and some of the remaining ones of the ultrasonic elements 111 to 116 may transmit ultrasonic waves with a predetermined small time difference. The predetermined small time difference may be a short time period before ultrasonic waves are received after the ultrasonic waves are initially transmitted. In detail, if a first ultrasonic element transmits ultrasonic waves to at least one target region, the first ultrasonic element may receive ultrasonic waves reflected from the target region according to the transmitted ultrasonic waves, or ultrasonic waves according to a radiation force generated from the target region or materials around the target region. A predetermined short time period may be set such that another ultrasonic element transmits ultrasonic waves before a first ultrasonic element receives the ultrasonic waves in response to transmitting the ultrasonic waves.

The frequencies of ultrasonic waves that are generated by the individual ultrasonic elements 111 to 116 may range from several kHz to several tens of kHz.

According to an exemplary embodiment, the ultrasonic elements 111 to 116 may generate different ultrasonic frequencies in units of groups. For example, ultrasonic frequencies $\lambda_1$, $\lambda_3$, and $\lambda_5$ that are generated by the odd-numbered ultrasonic elements, for example, the first ultrasonic element 111, the third ultrasonic element 113, and the fifth ultrasonic element 115 may be different from ultrasonic frequencies $\lambda_2$, $\lambda_4$, and $\lambda_6$ that are generated by the even-numbered ultrasonic elements, for example, the second ultrasonic element 112, the fourth ultrasonic element 114, and the sixth ultrasonic element 116. For example, the ultrasonic frequencies $\lambda_1$, $\lambda_3$, and $\lambda_5$ that are generated by the odd-numbered ultrasonic elements may have the same first frequency, and the ultrasonic frequencies $\lambda_2$, $\lambda_4$, and $\lambda_6$ that are generated by the even-numbered ultrasonic elements may have the same second frequency, different from or the same as the first frequency.

The ultrasonic waves generated by the ultrasonic array 110 may be transmitted to a plurality of different target regions t1 to t5 inside the object 98, as shown in FIG. 1. The ultrasonic waves generated by the individual ultrasonic elements 111 to 116 may be transmitted to the plurality of different target regions t1 to t5 at the same time, and may be focused on the plurality of target regions t1 to t5 at the same time. Accordingly, the ultrasonic probe 100 may perform multi-focusing to the plurality of target regions t1 to t5.

According to an exemplary embodiment, the ultrasonic array 110 may transmit ultrasonic waves such that the ultrasonic waves are focused on a plurality of target regions, for example, to the odd-numbered target regions t1, t3, and t5 spaced at predetermined intervals. For example, the individual ultrasonic elements 111 to 116 of the ultrasonic array 110 may transmit ultrasonic waves such that the transmitted ultrasonic waves are focused on the odd-numbered target regions, for example, the first target region t1, the third target region t3, and the fifth target region t5 spaced at predetermined intervals among the plurality of target regions t1 to t5, and not focused on the even-numbered target regions, for example, the second target region t2 and the fourth target region t4 interposed between the odd-numbered target regions t1, t3, and t5. The distances between the target regions t1, t3, and t5 on which the ultrasonic waves are focused may be appropriately determined by a user.

The ultrasonic array 110 may transmit the ultrasonic waves to the target regions t1 to t5 several times. For example, according to an exemplary embodiment, the ultrasonic array 110 may focus the ultrasonic waves on different target regions whenever transmitting the ultrasonic waves. For example, the ultrasonic array 110 may transmit ultrasonic waves such that the ultrasonic waves are focused on some of the plurality of target regions t1 to t5, for example, on the odd-numbered target regions t1, t3, and t5, and then again transmit ultrasonic waves such that the ultrasonic waves are focused on the remaining target regions of the plurality of target regions t1 to t5, for example, on the even-numbered target regions t2 and t4.

The ultrasonic waves transmitted to the predetermined target regions t1 to t5 may be reflected from the predetermined target regions t1 to t5. Or, the predetermined target regions t1 to t5 may vibrate according to the transmitted ultrasound waves to generate predetermined ultrasonic waves. Ultrasonic waves of frequencies that are different from two or more different frequencies reflected from or vibrated by the predetermined target regions t1 to t5 may be received by the ultrasonic array 110.

According to an exemplary embodiment, pulse current of a predetermined frequency may be applied only to a part (for example, the first to third ultrasonic elements 111 to 113) of the plurality of ultrasonic elements 111 to 116 of the ultrasonic array 110 so that only the first to third ultrasonic elements 111 to 113 to which the pulse current has been applied can generate ultrasonic waves of a predetermined frequency. For example, the ultrasonic waves generated by the ultrasonic elements 111 to 113 may be transmitted to the predetermined target regions t1 to t5. Then, the ultrasonic waves generated by the ultrasonic elements 111 to 113 may be reflected from the target regions t1 to t5, or materials of the target regions t1 to t5 may vibrate according to the ultrasonic waves generated by the ultrasonic elements 111 to 113 to generate ultrasonic waves. According to an exemplary embodiment, ultrasonic waves of a frequency that is different from two or more different frequencies of ultrasonic waves reflected from the target regions t1 to t5 or the transmitted ultrasonic waves may be received by all of the ultrasonic elements 111 to 116 or by some of the ultrasonic elements 111 to 116. As described above, all of the frequencies generated by the ultrasonic elements 111 to 116 may be same or the frequencies generated by the ultrasonic elements 111 to 116 may be different each other. Furthermore, only some of the frequencies generated by the ultrasonic elements 111 to 116 may be same or different.

The plurality of ultrasonic elements 111 to 116 of the ultrasonic array 110 may generate a plurality of transmission signals that overlap at different focusing locations, at the same time. Accordingly, multi-focusing using a plurality of locations in an object as focal points is possible.

Specifically, the ultrasonic array 110 may enable some of the plurality of ultrasonic elements 111 to 116 to transmit ultrasonic waves using a first location as a focal point, and the other ones of the plurality of ultrasonic elements 111 to 116 to transmit ultrasonic waves using a second location that is different from the first location, as a focal point. In this way, the ultrasonic array 110 may perform multi-focusing using a plurality of locations in an object as focal points.

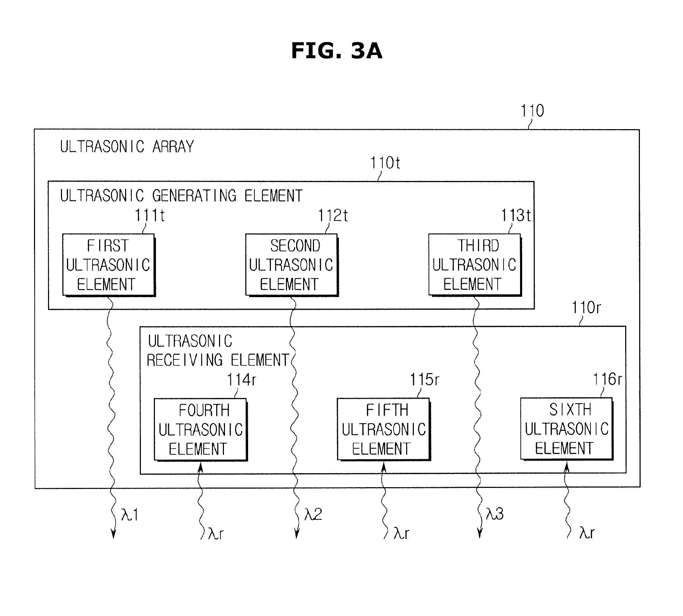

FIG. 3A is a block diagram illustrating a configuration of an ultrasonic array according to an exemplary embodiment of the present disclosure. As shown in FIG. 3A, the ultrasonic array 110 may include at least one ultrasonic generating element 110t to generate ultrasonic waves, and at least one ultrasonic receiving element 110r to receive ultrasonic waves. The ultrasonic generating element 110t may include first to third ultrasonic elements 111t, 112t, and 113t, and the ultrasonic receiving element 110r may include fourth to sixth ultrasonic elements 114r, 115r, and 116r, as shown in FIG. 3A.

The ultrasonic generating element 110t may generate ultrasonic waves of predetermined frequencies $\lambda_1$ to $\lambda_3$ according to predetermined pulse current. As described above, all of the frequencies $\lambda_1$ to $\lambda_3$ of ultrasonic waves generated by the first to third ultrasonic generating elements 111t to 113t may be the same or different.

As described above, the ultrasonic generating element 110t may generate a plurality of transmission signals that overlap at different focusing locations, at the same time. As a result, multi-focusing using a plurality of locations in an object as focal points may be performed. The ultrasonic generating element 110t may enable one or more of the first to third ultrasonic generating elements 111t to 113t to transmit ultrasonic waves using a focal point of a location that is different from that used by the other ones of the first to third ultrasonic generating elements 111 to 113t. In this way, the ultrasonic generating element 110t may perform multi-focusing. The ultrasonic waves of the predetermined frequencies $\lambda_1$ to $\lambda_3$ may be transmitted to the predetermined target regions t1 to t5 as shown in FIG. 1.

The ultrasonic receiving element 110r may receive ultrasonic waves of a predetermined frequency $\lambda_r$ reflected from the target regions t1 to t5 or generated by a radiation force of the internal tissue of the corresponding object. The ultrasonic receiving element 110r may convert the received ultrasonic waves into electrical signals corresponding to the received ultrasonic waves.

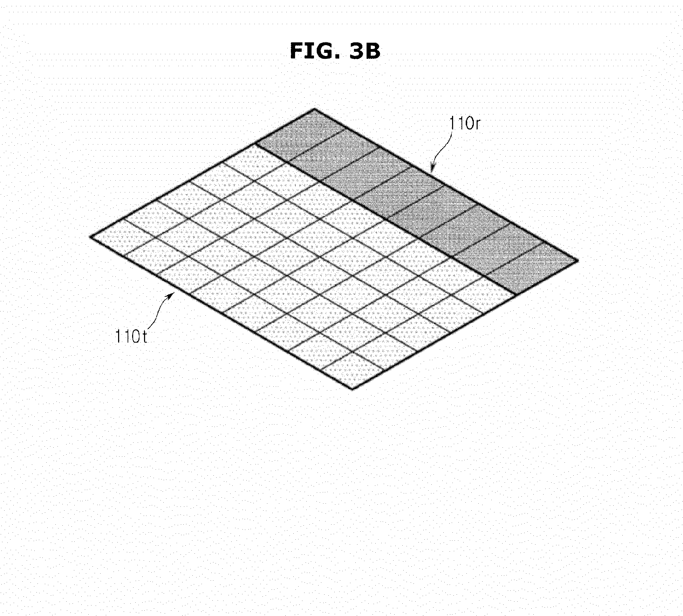

According to an exemplary embodiment, as shown in FIG. 3B, the ultrasonic generating element 110t may be a two-dimensional (2D) array transducer, and the ultrasonic receiving element 110r may be a one-dimensional (1D) array transducer. According to another exemplary embodiment, both the ultrasonic generating element 110t and the ultrasonic receiving element 110r may include 2D array transducers.

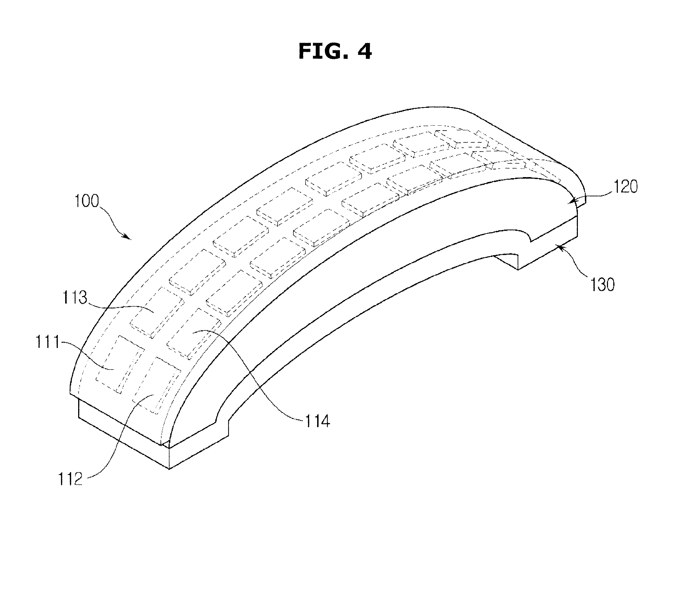

FIG. 4 is a perspective view illustrating the ultrasonic array 110 and the frame 120, according to an exemplary embodiment. As shown in FIG. 4, one or more ultrasonic elements 111 to 114 of the ultrasonic array 110 may be arranged on at least one side of the frame 120. Specifically, the ultrasonic elements 111 to 114 may be arranged in a predetermined pattern on the frame 120. For example, the ultrasonic elements 111 to 114 may be arranged in a plurality of rows on the frame 120, as shown in FIG. 4. Although not shown in FIG. 4, the ultrasonic elements 111 to 114 may be arranged in a zigzag pattern on the frame 120. The ultrasonic array 110 may be fixed on the frame 120, by using an adhesive, for example, an epoxy resin adhesive. However, for bonding and fixing of the ultrasonic array 110 with the frame 120, any other kind of coupling, fixing, and bonding means may be used.

The frame 120 may include a resting groove or a protrusion formed in a side on which the ultrasonic array 110 can be appropriately arranged. The ultrasonic array 110 may be arranged on a groove or protrusion of a predetermined pattern.

As shown in FIG. 4, a substrate 130 to control current that is applied to the ultrasonic array 110 or receive the electrical signals that are output from the ultrasonic array 110 may be provided on the other side of the frame 120. According to an exemplary embodiment, on the substrate 130 may be formed to include various circuitry to control the ultrasonic array 110.

The ultrasonic elements 111 to 114 may be ultrasonic transducers. The ultrasonic transducer may be a device for converting one form of energy into another form of energy. For example, the ultrasonic transducer may convert electric energy into wave energy or wave energy into electric energy. That is, the ultrasonic transducer may perform mutual conversion between wave energy and electric energy. The ultrasonic transducer may be a magnetostrictive ultrasonic transducer that converts wave energy into electric energy using the magnetostrictive effect of a magnetic material, a piezoelectric ultrasonic transducer using the piezoelectric effect of a piezoelectric material, or a capacitive micromachined ultrasonic transducer (CMUT) that transmits and receives ultrasonic waves using vibration of several hundreds or thousands of micromachined thin films. However, the ultrasonic transducer may be any other type of the ultrasonic transducer capable of generating ultrasonic waves according to electrical signals or generating electrical signals according to ultrasonic waves.

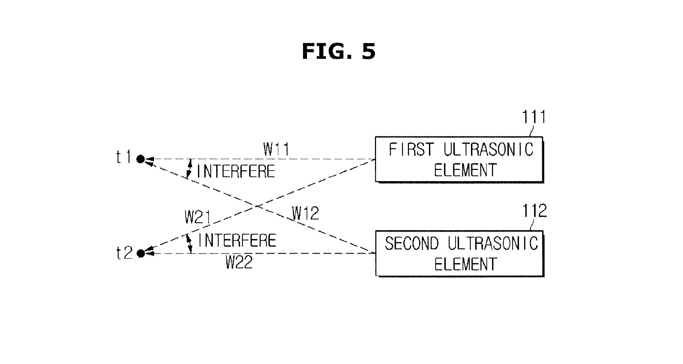

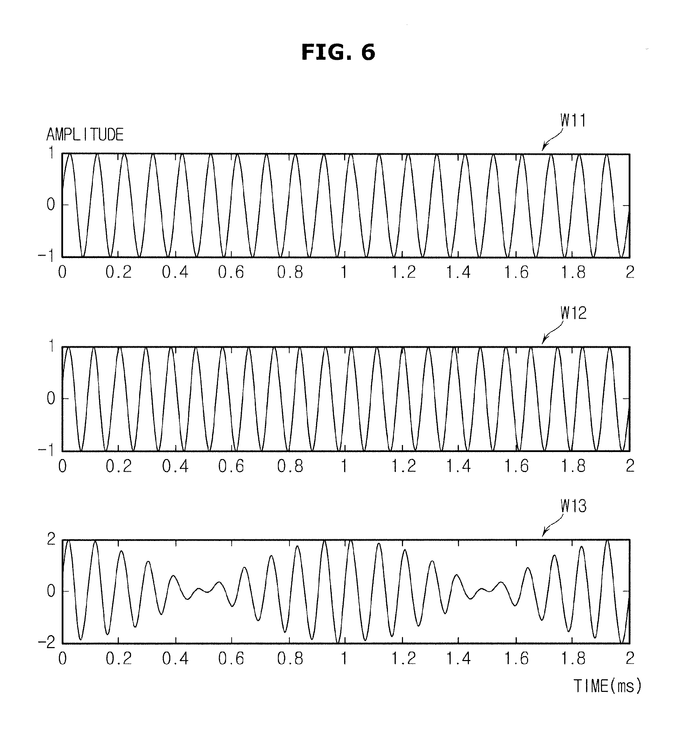

FIG. 5 is a view for describing interference between ultrasonic waves of different frequencies. FIG. 6 is a view for describing the beating. As described above, all or some of the frequencies $\lambda_1$ to $\lambda_6$ of ultrasonic waves generated by the individual ultrasonic elements 111 to 116 of the ultrasonic array 110 or by the ultrasonic elements 111t to 113t may be different from one another. For example, the ultrasonic waves of the different frequencies $\lambda_1$ to $\lambda_6$ may interfere so that the target regions t1 to t5 may be influenced by the results of the interference by the different frequencies $\lambda_1$ to $\lambda_6$. For example, the ultrasonic waves of the different frequencies $\lambda_1$ to $\lambda_6$ may interfere to generate interference ultrasonic waves of a predetermined frequency, and the interference ultrasonic waves may arrive at the target regions t1 to t5 to be reflected from the target regions t1 to t5 or to vibrate the target regions t1 to t5.

For example, as shown in FIG. 5, if the first ultrasonic element 111 generates first ultrasonic waves w11 and w21 of a first frequency $\lambda_1$, and the second ultrasonic element 112 generates second ultrasonic waves w12 and w22 of a second frequency $\lambda_2$ that is different from the first frequency $\lambda_1$, the first ultrasonic waves w11 and w21 of the first frequency $\lambda_1$ and the second ultrasonic waves w12 and w22 of the second frequency $\lambda_2$ may interfere to generate new composite waves, that is, interference ultrasonic waves. The frequency of the generated interference waves may be different from the frequencies $\lambda_1$ and $\lambda_2$ of the first and second ultrasonic waves w11 and w22. As shown in FIG. 6, the second frequency $\lambda_2$ of the second ultrasound wave w12 is slightly greater than the first frequency $\lambda_1$ of the first ultrasonic wave w11. The resultant interference ultrasonic wave w13 shows periodic increases and decreases in amplitude. The interference ultrasonic waves may arrive at respective target regions t1 and t2, and apply vibration to the respective target regions t1 and t2 or be reflected from the respective target regions t1 and t2. The first and second ultrasonic waves w11 and w22 generated by the first and second ultrasonic elements 111 and 112 can be respectively expressed as Equations (1) and (2), below.

$$\Psi_1 = A\sin(2\pi f_1 t), \text{ and} \tag{1}$$

$$\Psi_2 = A\sin(2\pi f_2 t). \tag{2}$$

In Equations (1) and (2), $\psi_1$ represents the first ultrasonic wave w11 or w21, $\psi_2$ represents the second ultrasonic wave w12 or w22, $f_1$ and $f_2$ represent first and second frequencies $\lambda_1$ and $\lambda_2$, t represents time, and A is a constant. The interference ultrasonic waves can be expressed as Equation (3), below.

$$\Psi = \Psi_1 + \Psi_2 = 2A\cos(2\pi\frac{f_1-f_2}{2}t)\sin(2\pi\frac{f_1+f_2}{2}t), \tag{3}$$

where $\psi$ represents interference ultrasonic waves appearing when the first and second ultrasonic waves w11 to w22 interfere with each other. The frequency and amplitude of the interference ultrasonic waves may be different from those of the first and second ultrasonic waves w11 to w22 generated by the first and second ultrasonic elements 111 and 112. Generally, the frequency of the interference ultrasonic waves may be lower than the frequencies of the first and second ultrasonic waves w11 to w22.

When the interference ultrasonic waves arrive at the target regions t1 and t2, ultrasonic waves may be generated from the target regions t1 and t2 or materials around the target regions t1 and t2, and the generated ultrasonic waves may be received by the ultrasonic array 110. For example, if the interference ultrasonic waves arrive at the target regions t1 and t2, the target regions t1 and t2 may be subject to a radiation force by the interference ultrasonic waves to generate vibration waves at a frequency corresponding to the frequency of the interference ultrasonic waves. The vibration waves may be received and collected by the ultrasonic array 110.

As shown in FIG. 1, according to an exemplary embodiment, the ultrasonic probe 110 may further include a lens 140 to cover the outer side of the frame 120 on which the ultrasonic array 110 is mounted, a housing 150 to accommodate various components of the ultrasonic probe 110, and communication means 160, i.e., a cable or a wireless connection, to transmit data.

The lens 140 may cover the ultrasonic elements 111 to 116 of the ultrasonic array 110 and related components to prevent the ultrasonic elements 111 to 116 from directly contacting the outside, in order to protect the ultrasonic elements 111 to 116. The lens 140 may allow ultrasonic waves received from the outside to be appropriately transferred to the individual ultrasonic elements 111 to 116. The lens 140 may have a curved shape. According to an exemplary embodiment, the lens 140 of the ultrasonic probe 100 may be an acoustic lens.

The housing 150 may accommodate various components of the ultrasonic probe 100 to fix or protect the various components. More specifically, the housing 150 may stably fix various components, such as the ultrasonic array 110, the frame 120 on which the ultrasonic array 110 is arranged, a circuit substrate provided on the rear side of the frame 120, and the lens 140, or the housing 150 may prevent the various components such as the circuit substrate from being exposed to the outside. Although not shown in the drawings, the housing 150 may further include a handle for allowing a user to conveniently manipulate the housing 150. Also, in the outer side of the housing 150 may be provided an input device, such as various buttons, a touch screen, or a trackball, for enabling a user to control the ultrasonic probe 100.

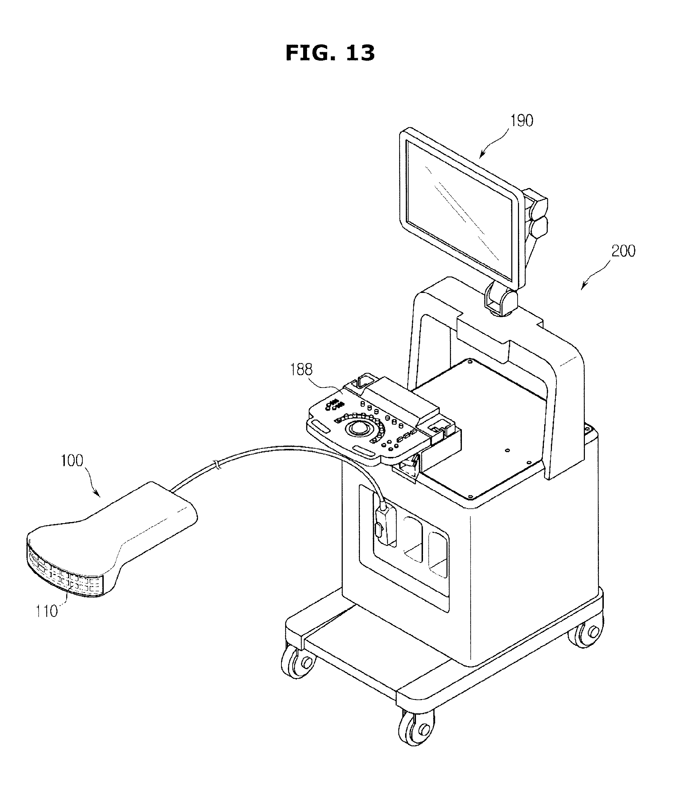

The communication means 160 may transmit data about ultrasonic waves collected by the ultrasonic probe 100 to a main body 200 (see FIG. 13) of an external ultrasonic imaging apparatus. According to an exemplary embodiment, the communication means 160 may transfer electrical signals acquired by converting ultrasonic waves, digital electrical signals obtained by performing analog-to-digital conversion on the acquired electrical signals, or other electrical signals acquired using the electrical signals, to the main body 200. For example, the communication means 160 may be a cable, as shown in FIG. 1. As another example, the communication means 160 may be a wireless communication module. The wireless communication module can transmit and receive data based on various mobile communication standards including Bluetooth™, Wireless Fidelity (WiFi), 3rd Generation Partnership Project (3GPP), 3GPP2, and/or Worldwide Interoperability for Microwave Access (WiMax).

According to an exemplary embodiment, the ultrasonic probe 100 may further include an ultrasonic signal obtainer 170 to receive at least one electrical signal output from the ultrasonic array 110, as shown in FIG. 2. The ultrasonic signal obtainer 170 may receive electrical signals of a plurality of channels from the individual ultrasonic elements, for example, the first to sixth ultrasonic elements 111 to 116 of the ultrasonic array 110, and acquire ultrasonic signals for the plurality of target regions t1 to t5 based on the received electrical signals of the plurality of channels.

The ultrasonic signal obtainer 170 may acquire ultrasonic signals for the plurality of target regions t1 to t5, based on the plurality of ultrasonic waves w11 to w22 transmitted from the ultrasonic array 110 or ultrasonic waves received by the ultrasonic array 110. More specifically, the ultrasonic signal obtainer 170 may acquire ultrasonic signals for the plurality of target regions t1 to t5, based on the frequencies $\lambda_1$ to $\lambda_6$ of the transmitted ultrasonic waves w11 to w22 and the frequency $\lambda_r$ of the received ultrasonic waves. The ultrasonic signal obtainer 170 may acquire ultrasonic signals for the respective target regions t1 to t5 independently. For example, the plurality of target regions t1 to t5 may be spaced at predetermined intervals. According to an exemplary embodiment, the ultrasonic signal obtainer 170 may first acquire ultrasonic signals for predetermined target regions, for example, the odd-numbered target regions t1, t3, and t5 among the plurality of target regions t1 to t5, store the ultrasonic signals, successively acquire ultrasonic signals for other target regions, for example, the even-numbered target regions t2 and t4, and then combine the acquired ultrasonic signals to acquire ultrasonic signals for all of the target regions t1 to t5. As another example, the ultrasonic signal obtainer 170 may acquire ultrasonic signals based on ultrasonic signals transferred from all of the ultrasonic elements 111 to 116 of the ultrasonic array 110, or the ultrasonic signal obtainer 170 may acquire ultrasonic signals based on ultrasonic signals transferred from predetermined ultrasonic elements of the ultrasonic array 110.

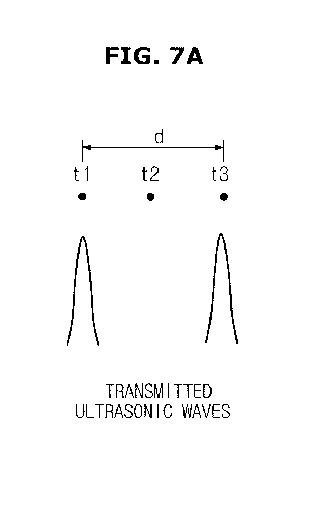

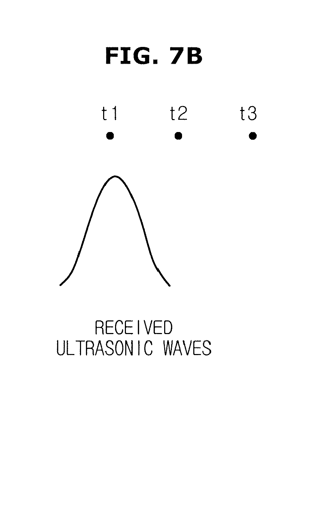

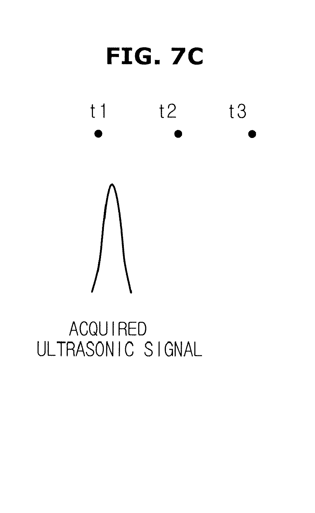

FIGS. 7 to 12 are views for describing acquisition of ultrasonic signals according to an exemplary embodiment. As shown in FIG. 7A, when the ultrasonic array 110 generates a plurality of ultrasonic waves, the generated ultrasonic waves may be focused on predetermined target regions, for example, first and third target regions t1 and t3 of a plurality of target regions t1 to t3. FIG. 7A shows shapes of ultrasonic beams focused on the respective target regions t1 and t3. For example, the ultrasonic waves focused on the predetermined target regions, for example, the first and third target regions t1 and t3 may be ultrasonic waves that have been transmitted at the same time. If distances between the ultrasonic elements 111 to 116 and the predetermined target regions, for example, the first and third target regions t1 and t3 are nearly the same and ultrasonic waves transmitted from the ultrasonic elements 111 to 116 pass through nearly the same materials until arriving at the first and third target regions t1 and t3, most of the plurality of ultrasonic waves that have been transmitted at the same time may be focused on the predetermined target regions, for example, the first and third target regions t1 and t3.

According to an exemplary embodiment, the ultrasonic waves focused on the predetermined target regions t1 and t3 may be interference ultrasonic waves generated according to interference between a plurality of ultrasonic waves of different frequencies. That is, the ultrasonic array 110 may generate a plurality of ultrasonic waves of different frequencies for interference ultrasonic waves focused on the predetermined target regions, for example, the first and third target regions t1 and t3 of the plurality of target regions t1 to t3.

According to an exemplary embodiment, the predetermined target regions, for example, the first and third target regions t1 and t3 on which the plurality of ultrasonic waves are focused may be spaced by a predetermined distance d.

As described above, if ultrasonic waves are focused on the target regions t1 and t3, the target regions t1 and t3 on which the ultrasonic waves have been focused may vibrate according to the frequencies of the focused ultrasonic waves, and generate vibration waves, for example, ultrasonic waves in the shape of a beam, as shown in FIG. 7B. For example, the frequency of the ultrasonic waves generated from the target regions t1 and t3 may be relatively lower than the frequency of the ultrasonic waves focused on the target regions t1 and t3. For example, as shown in FIGS. 7A and 7B, ultrasonic waves focused on the target regions t1 and t3 may be high-frequency ultrasonic waves, and ultrasonic waves generated from the target regions t1 and t3 may be low-frequency ultrasonic waves. The generated ultrasonic waves may include harmonic components. The harmonic components of the generated ultrasonic waves may be second harmonic components or third harmonic components. Also, the harmonic components may include other harmonic components.

The ultrasonic signal obtainer 170 may receive electrical signals obtained by converting the ultrasonic waves generated from the target regions t1 and t3, from the ultrasonic elements 111 to 116, and acquire ultrasonic signals for a predetermined target region, for example, the first target region t1, based on the ultrasonic waves (see FIG. 7B) generated from the target regions t1 and t3 and the ultrasonic waves (see FIG. 7A) focused on the target regions t1 and t3. The ultrasonic signals may include harmonic components. The harmonic components of the ultrasonic signals may include all harmonic components generated or increased while passing through the medium, and the harmonic components included in the ultrasonic waves generated from the target regions t1 and t3.

For example, the ultrasonic signal obtainer 170 may synthesize ultrasonic waves focused on a predetermined target region, for example, the first target region t1 with ultrasonic waves generated from the first target region t1 to generate an ultrasonic signal having a shape of an ultrasonic beam, as shown in FIG. 7C. As a result, an appropriate ultrasonic signal for the first target region t1 may be acquired.

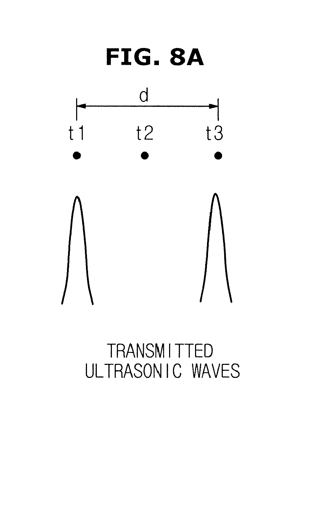

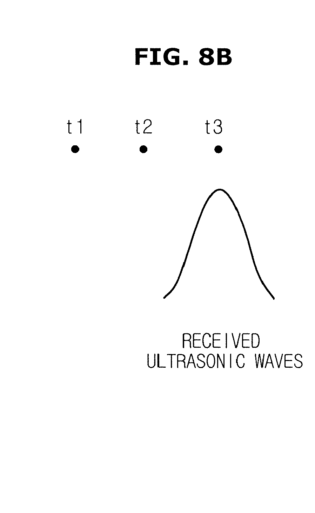

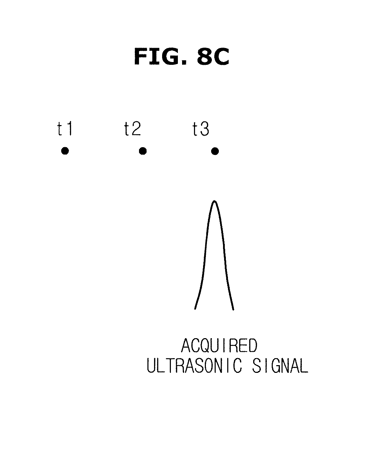

Likewise, as shown in FIGS. 8A, 8B, and 8C, the ultrasonic signal obtainer 170 may acquire an appropriate ultrasonic signal for the third target region t3 among the plurality of target regions t1 to t3. Ultrasonic waves focused on the third target region t3 may be generated and transmitted at the same time as ultrasonic waves focused on the first target region t1.

Accordingly, the ultrasonic signal obtainer 170 may acquire ultrasonic signals for predetermined target regions, for example, an ultrasonic signal for the first target region t1 and an ultrasonic signal for the third target region t3.

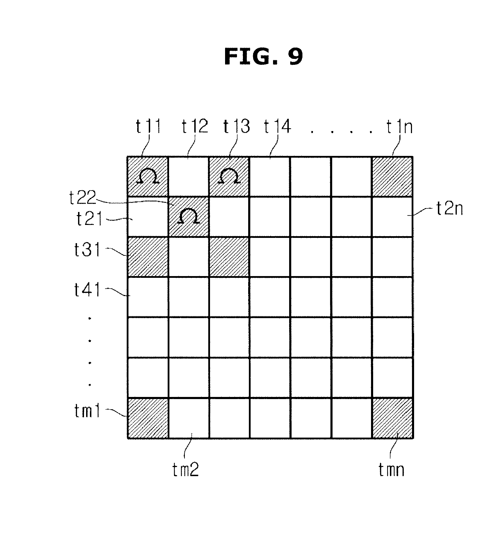

As shown in FIG. 9, the ultrasonic signal obtainer 170 may acquire ultrasonic signals for a plurality of target regions t11 to tmn. More specifically, the ultrasonic array 110 may transmit ultrasonic waves such that ultrasonic waves or interference ultrasonic waves are focused on the plurality of target regions t11 to tmn spaced at predetermined intervals, and the ultrasonic signal obtainer 170 may acquire ultrasonic signals for the plurality of target regions t11 to tmn. For example, the ultrasonic signal obtainer 170 may transmit ultrasonic waves such that ultrasonic waves or interference ultrasonic waves are not focused at the same time on neighboring target regions, for example, the eleventh target region t11 and the twelfth target region t12 or the twenty first target region t21, whereas ultrasonic waves are focused at the same time on target regions spaced by a predetermined distance, for example, the eleventh target region t11 and the thirteenth target region t13 or the twenty second target region t22. The ultrasonic signal obtainer 170 may generate ultrasonic signals for the target regions t11, t13, and t22 on which ultrasonic waves transmitted by the ultrasonic array 110 are focused at the same time.

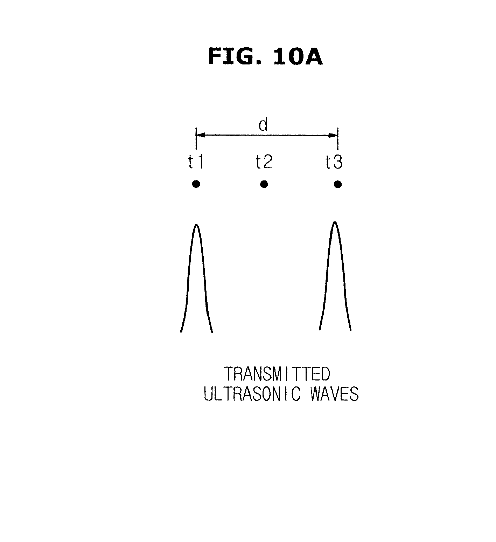

According to an exemplary embodiment, as shown in FIG. 10A, the ultrasonic array 110 may generate and transmit ultrasonic waves that are focused on predetermined target regions, for example, the first and third target regions t1 and t3 among the plurality of target regions t1 to t3, and then generate a plurality of ultrasonic waves of different frequencies for ultrasonic waves or interference ultrasonic waves that are focused on the other target region, for example, the second target region t2 among the plurality of target regions t1 to t3, on which the ultrasonic waves have been not focused.

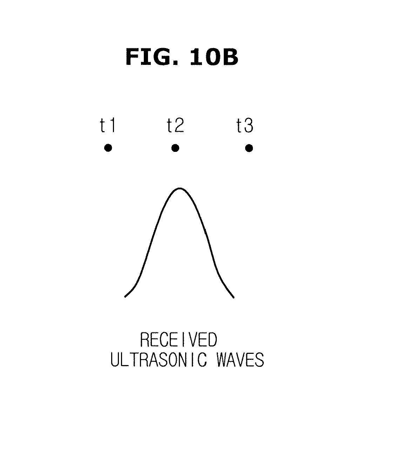

Due to the ultrasonic waves generated by the ultrasonic array 110 or the interference ultrasonic waves according to interference between the ultrasonic waves generated by the ultrasonic array 110, the other target region, for example, the second target region t2 on which the ultrasonic waves have been not focused may vibrate to generate ultrasonic waves as shown in FIG. 10B.

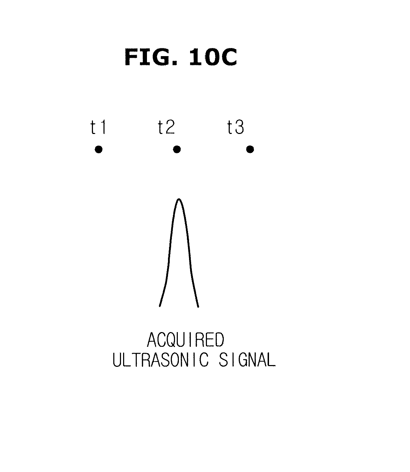

The ultrasonic signal obtainer 170 may receive electrical signals obtained by converting ultrasonic waves generated from the second target region t2, from the ultrasonic elements 111 to 116, and acquire an ultrasonic signal for the second target region t2, based on the ultrasonic waves (see FIG. 10B) generated from the second target region t2 and the ultrasonic waves (see FIG. 10A) focused on the target region t2. In the same way as described above, the ultrasonic signal obtainer 170 may synthesize the ultrasonic waves focused on the second target region t2 with the ultrasonic waves generated from the second target region t2 to generate an ultrasonic signal having a predetermined shape of an ultrasonic beam, as shown in FIG. 10C. As a result, an appropriate ultrasonic signal for the second target region t2 may be acquired.

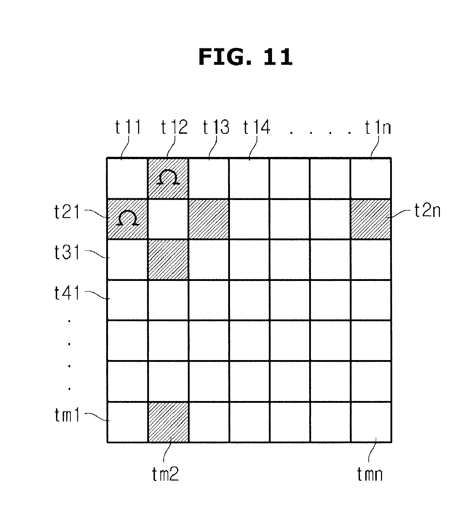

As shown in FIG. 11, the ultrasonic signal obtainer 170 may acquire ultrasonic signals for a plurality of target regions, for example, the twelfth target region t12, which has not been acquired in the process described above with reference to FIG. 9. Specifically, the ultrasonic array 110 may transmit ultrasonic waves such that ultrasonic waves or interference ultrasonic waves are focused on predetermined target regions, for example, the twelfth target region t12 and the twenty first target region t21, spaced by a predetermined distance, from which no ultrasonic signal has been acquired, and the ultrasonic signal obtainer 170 may acquire ultrasonic signals for the target regions t12 and t21 on which the ultrasonic waves transmitted by the ultrasonic array 110 have been focused.

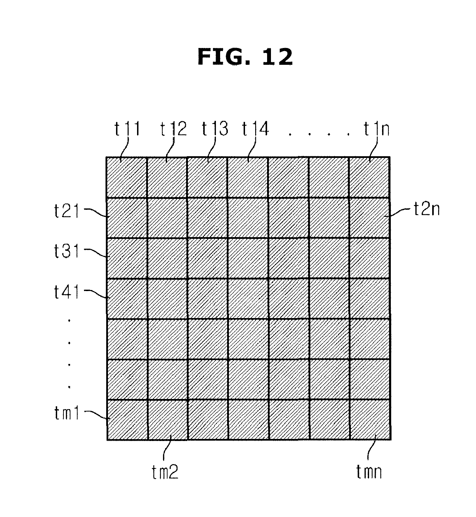

The ultrasonic signal obtainer 170 may first acquire ultrasonic signals for first target regions, and successively acquire ultrasonic signals for second target regions, thereby acquiring ultrasonic signals for all of the plurality of target regions t11 to tmn, as shown in FIG. 12.

According to an exemplary embodiment, the ultrasonic receiving element 110r (see FIG. 3A) receives a multi-array ultrasonic signal, and, thus, may distinguish and receive signals from different target regions although the signals overlap. Accordingly, by transmitting ultrasonic waves for the second target regions before acquiring ultrasonic signals for the first target regions, processing speed may increase.

The ultrasonic signals for the respective target regions, for example, the first to third target regions t1 to t3 may be synthesized to be used as raw data for generating an ultrasonic image.

The described-above relates to an example of acquiring ultrasonic signals by transmitting a plurality of ultrasonic waves two times to different target regions, for example, the odd-numbered target regions t1, t3, and t5 and the even-numbered target regions t2 and t4 and receiving ultrasonic waves two times from the target regions t1 to t5. However, according to an exemplary embodiment, it is also possible to acquire ultrasonic signals by transmitting a plurality of ultrasonic waves more times and receiving ultrasonic waves by the number of times corresponding to the number of times of transmission. For example, the plurality of target regions t1 to t5 may be grouped into three groups, a plurality of ultrasonic waves may be transmitted to the respective groups to collect ultrasonic waves for the respective groups, and then ultrasonic signals for the plurality of target regions t1 to t5 may be acquired based on the ultrasonic waves transmitted to the respective groups and the ultrasonic waves collected from the respective groups. However, it will be also possible to group the plurality of target regions t1 to t5 into more groups, to transmit a plurality of ultrasonic waves to the respective groups to collect ultrasonic waves for the respective groups, and then to acquire ultrasonic signals for the plurality of target regions t1 to t5 based on the ultrasonic waves transmitted to the respective groups and the ultrasonic waves collected from the respective groups.

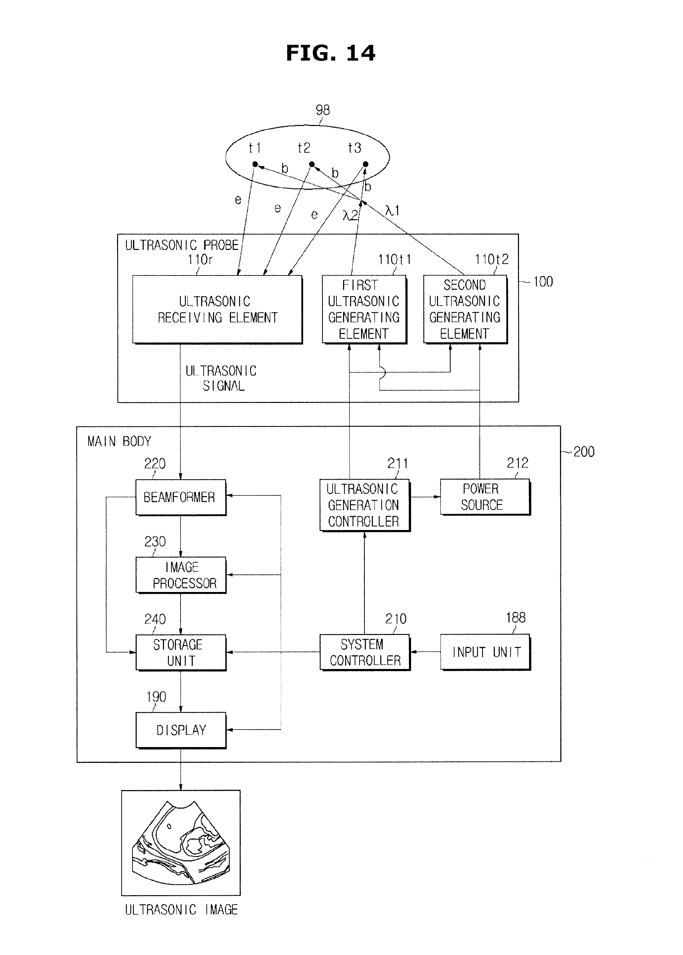

The ultrasonic signals acquired by the ultrasonic obtainer 170 may be transferred to the main body 200 (see FIGS. 13 and 14) of the ultrasonic imaging apparatus through the communication means 160, etc., or transferred to the focuser 180 in the ultrasonic probe 100.

For example, the focuser 180 may focus electrical signals of a plurality of channels output from the individual ultrasonic elements 111 to 116 of the ultrasonic array 110, or ultrasonic signals of a plurality of channels acquired by the ultrasonic signal obtainer 170.

According to an exemplary embodiment, the focuser 180 may correct time differences between the ultrasonic signals of the individual channels, caused since ultrasonic waves generated from the same target regions t1 to t6 arrive at the respective ultrasonic elements 111 to 116 at different times, and focus the ultrasonic waves of the plurality of channels subjected to the time-difference correction to generate a beamformed ultrasonic signal $z_0$. The beamformed ultrasonic signal $z_0$ may be transferred to the main body 200 of the ultrasonic imaging apparatus through the communication means 160, etc., or to the ultrasonic signal obtainer 170.

If the ultrasonic signal obtainer 170 receives the beamformed ultrasonic signal $z_0$ from the focuser 180, the ultrasonic signal obtainer 170 may acquire an ultrasonic signal based on the ultrasonic signals transmitted to the target regions t1 to t3 and the beamformed ultrasonic signal $z_0$. The beamformed ultrasonic signal $z_0$ may be one of the ultrasonic signals shown in FIGS. 7B, 8B, and 10B.

The focuser 180 may focus the ultrasonic signals acquired by the ultrasonic signal obtainer 170, that is, the ultrasonic signals shown in FIGS. 7C, 8C, and 10C to thereby generate a beamformed ultrasonic signal z. The beamformed ultrasonic signal z may be transferred to the main body 200 of the ultrasonic imaging apparatus through the communication means 160, etc.

Hereinafter, the ultrasonic imaging apparatus may be described with reference to FIGS. 13 to 16. FIG. 13 is a perspective view for describing an ultrasonic imaging apparatus according to an exemplary embodiment, and FIG. 14 is a block diagram illustrating a configuration of an ultrasonic imaging apparatus according to an exemplary embodiment. As shown in FIGS. 13 and 14, the ultrasonic imaging apparatus may include the ultrasonic probe 100 and the main body 200.

The ultrasonic probe 100 may collect a plurality of ultrasonic waves generated from an object 98. The ultrasonic probe 100 may be an ultrasonic probe as shown in FIG. 1. The ultrasonic probe 100 may include the ultrasonic array 110 including a plurality of ultrasonic elements, as shown in FIG. 14.

According to an exemplary embodiment, the ultrasonic probe 100 may be a unitary ultrasonic probe capable of transmitting and receiving ultrasonic waves, or a combination ultrasonic probe in which an ultrasonic transmission probe is combined with an ultrasonic reception probe.

According to an exemplary embodiment, the ultrasonic array 110 may generate ultrasonic waves of a predetermined frequency according to alternating current that is applied from a power source 212 in the main body 200 to the individual ultrasonic elements, and transmit the generated ultrasonic waves to the target regions t1 to t3 inside the object 98. The ultrasonic array 110 may generate ultrasonic waves of a plurality of different frequencies $\lambda_1$ and $\lambda_2$, and transmit the ultrasonic waves of the different frequencies $\lambda_1$ and $\lambda_2$ to the target regions t1 to t3 inside the object 98. For example, the ultrasonic array 110 may enable a group of the ultrasonic elements to generate and transmit ultrasonic waves of the first frequency $\lambda_1$ and another group of the ultrasonic elements to generate and transmit ultrasonic waves of the second frequency $\lambda_2$. If the ultrasonic array 110 generates the ultrasonic waves of the different frequencies $\lambda_1$ and $\lambda_2$ and transmits the ultrasonic waves of the different frequencies $\lambda_1$ and $\lambda_2$ to the target regions t1 to t3 inside the object 98, the ultrasonic waves of the different frequencies $\lambda_1$ and $\lambda_2$ may interfere to generate interference ultrasonic waves b of a predetermined frequency, as expressed by Equation (3). If the interference ultrasonic waves b arrive at the individual target regions t1 to t3, the target regions t1 to t3 may generate ultrasonic waves e corresponding to the interference ultrasonic waves b.

The ultrasonic array 110 may transmit the ultrasonic waves of the plurality of different frequencies $\lambda_1$ and $\lambda_2$ to the target regions t1 to t3 inside the object 98, at the same time. In other words, the ultrasonic array 110 may focus the ultrasonic waves of the plurality of different frequencies $\lambda_1$ and $\lambda_2$ on the target regions t1 to t3 to transmit the ultrasonic waves to the target regions t1 to t3. The ultrasonic array 110 may transmit the ultrasonic waves of the plurality of different frequencies $\lambda_1$ and $\lambda_2$ several times to the target regions t1 to t3 inside the object 98. According to an exemplary embodiment, the ultrasonic array 110 may sequentially transmit the ultrasonic waves to different target regions. For example, the ultrasonic array 110 may transmit the ultrasonic waves to a predetermined target region, and then transmit the ultrasonic waves to another target region to which the ultrasonic waves have been not transmitted.

The ultrasonic array 110 may receive ultrasonic waves transferred from the target regions t1 to t3 inside the object 98, and convert the received ultrasonic waves into electrical signals, through the ultrasonic elements. The converted electrical signals may be transferred to a beamformer 220 of the main body 200.

According to an exemplary embodiment, the plurality of ultrasonic elements 110 may include one or more ultrasonic generating elements 110t1 and 110t2 and an ultrasonic receiving element 110r, as shown in FIG. 14. For example, the ultrasonic generating elements 110t1 and 110t2 may generate predetermined ultrasonic waves, and transmit the predetermined ultrasonic waves to the plurality of target regions t1 to t3 inside the object 98.

The ultrasonic generating elements 110t1 and 110t2 may generate ultrasonic waves of different frequencies $\lambda_1$ and $\lambda_2$. If the first and second ultrasonic generating elements 110t1 and 110t2 generate ultrasonic waves of different frequencies $\lambda_1$ and $\lambda_2$ and transmit the ultrasonic waves of the different frequencies $\lambda_1$ and $\lambda_2$ to the target regions t1 to t3 inside the object 98, the ultrasonic waves of the different frequencies $\lambda_1$ and $\lambda_2$ may interfere to generate interference ultrasonic waves b, and the respective target regions t1 to t3 may generate ultrasonic waves e corresponding to the interference ultrasonic waves b.

The ultrasonic receiving element 110r may collect the ultrasonic waves e generated by the target regions t1 to t3, convert the ultrasonic waves e into electrical signals, that is, ultrasonic signals, and output the ultrasonic signals. The ultrasonic signals output from the ultrasonic receiving element 110r may be transferred to the main body 200.

According to an exemplary embodiment, the ultrasonic probe 100 of the ultrasonic imaging apparatus may further include an ultrasonic signal obtainer 170 as shown in FIG. 2. The ultrasonic probe 100 may generate ultrasonic signals based on the transmitted ultrasonic waves and the received ultrasonic waves. The generated ultrasonic signals may be transferred to the beamformer 220 of the main body 200. The ultrasonic probe 100 may further include the focuser 180 as shown in FIG. 2. In this case, since the focuser 180 performs beamforming on ultrasonic waves to generate beamformed ultrasonic signals z and $z_0$, the beamformer 220 of the main body 200 does not need to perform beamforming.

Referring to FIG. 14, the main body 200 may include a system controller 210, an ultrasonic generation controller 211, an image processor 230, a storage unit 240, an input unit 188, and a display 190.

The system controller 210 may control overall operations of the main body 200 and/or the ultrasonic probe 100. The system controller 210 may generate an appropriate control command according to a predetermined setting or according to a user's instruction or command received through the input unit 188, and transfer the generated control command to the ultrasonic probe 100 or the individual components of the main body 200, thereby controlling overall operations of the ultrasonic imaging apparatus.

The system controller 210 may calculate and measure the velocity of transmitted ultrasonic waves, for example, the velocity of ultrasonic waves of a low frequency. The system controller 210 may use Doppler imaging to measure the velocity of transmitted ultrasonic waves. The Doppler imaging is to measure the velocity of ultrasonic waves by comparing a plurality of images acquired by retransmitting ultrasonic waves of a short wavelength and then beamforming. If the velocity of the ultrasonic waves is high, the system controller 210 may determine that a vibration level of the low frequency is high, and if the velocity of the ultrasonic waves is low, the system controller 210 may determine that a vibration level of the low frequency is low.

The system controller 210 may control the ultrasonic probe 100 to transmit pulse echo to an object. When pulse echo is transmitted, the movement may be measured by using the Doppler effect.

The ultrasonic generation controller 211 may receive a control command from the system controller 210, generate a predetermined control signal according to the received control command, and transfer the control signal to the ultrasonic array 110 or to the ultrasonic generating elements 110t1 and 110t2 of the ultrasonic array 110. The ultrasonic array 110 or the ultrasonic generating elements 110t1 and 110t2 may vibrate according to the received control signal to generate ultrasonic waves. The ultrasonic generation controller 211 may generate a predetermined control command for enabling the ultrasonic array 110 to transmit ultrasonic waves of a plurality of frequencies or the first and second ultrasonic generating elements 110t1 and t2 to transmit ultrasonic waves of different frequencies, and transfer the predetermined control command to the ultrasonic array 110 or to the first and second ultrasonic generating elements 110t1 and 110t2.

The ultrasonic generation controller 211 may generate another control signal for controlling the power source 212 electrically connected to the ultrasonic array 110, and transfer the control signal to the power source 212. The power source 212 may apply predetermined alternating current to the ultrasonic array 110 or to the ultrasonic generating elements 110t1 and 110t2, according to the control signal. Then, the ultrasonic array 110 or the ultrasonic generating elements 110t1 and 110t2 may vibrate according to the applied alternating current to generate ultrasonic waves, and transmit the ultrasonic waves to the target regions t1 to t3.

When the power source 212 applies alternating current to the ultrasonic generating elements 110t1 and 110t2, the power source 212 may apply different alternating currents to the first and second ultrasonic generating elements 110t1 and 110t2, respectively. Then, the first and second ultrasonic generating elements 110t1 and 110t2 may generate ultrasonic waves of different frequencies. The frequencies of alternating currents that are respectively applied to the first and second ultrasonic generating elements 110t1 and 110t2 may be determined by the ultrasonic generation controller 211.

The beamformer 220 of the main body 200 may receive ultrasonic signals transferred from the ultrasonic array 110 or the ultrasonic receiving element 110r, filter the received ultrasonic signals according to the reception frequency, and then generate beamformed ultrasonic signals based on the results of the filtering.

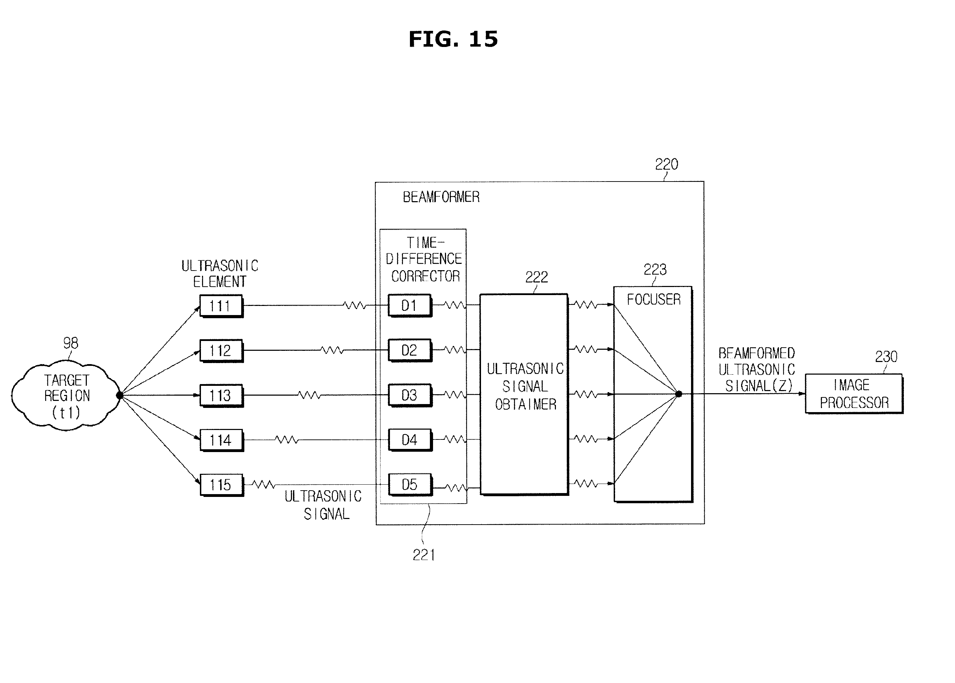

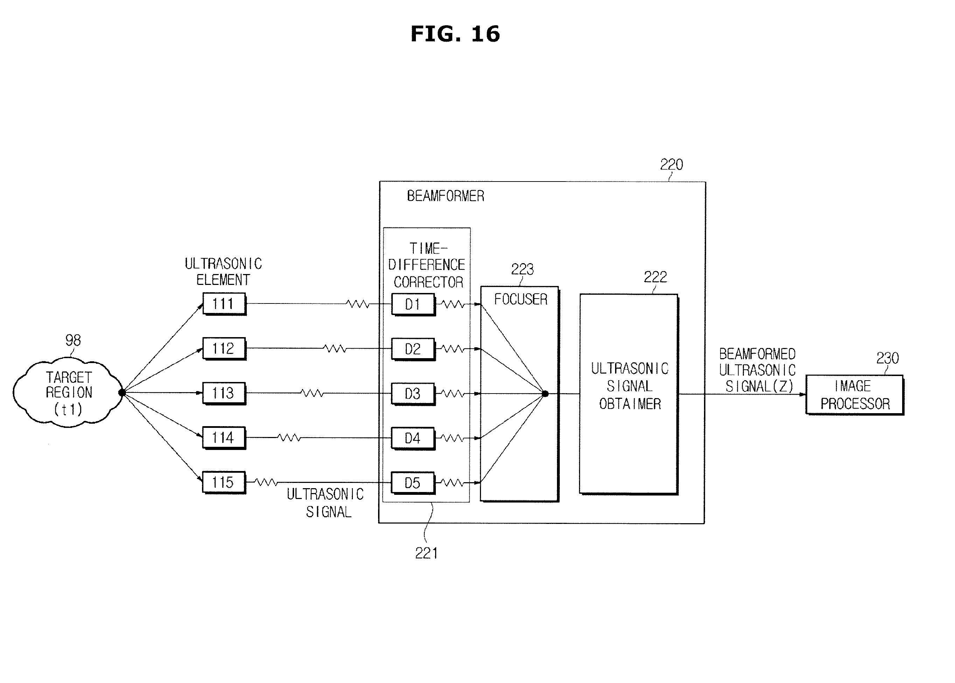

FIGS. 15 and 16 are block diagrams illustrating configurations of the beamformer 220 according to an exemplary embodiment.

As shown in FIGS. 15 and 16, the beamformer 220 may include a time-difference corrector 221, an ultrasonic signal obtainer 222, and a focuser 223.

Although not shown in FIGS. 15 and 16, the beamformer 220 may further include a filter to filter received signals. The filter may filter received ultrasonic signals using a predetermined filter according to a desired reception frequency. For example, if a reception frequency is a harmonic frequency, the filter may perform filtering according to the harmonic frequency. If a reception frequency is not a harmonic frequency, the filter may perform filtering according to the reception frequency.

The time-difference corrector 221 may correct time differences between ultrasonic signals received by a plurality of ultrasonic elements 111 to 115. Ultrasonic waves generated from a target region t1 in an object 98 may be received by the ultrasonic elements 111 to 115. The physical distances between the individual ultrasonic elements 111 to 115 and the target region t1 are different from each other; however, sound velocity of ultrasonic waves is nearly constant. Therefore, the respective ultrasonic elements 111 to 115 may receive ultrasonic waves generated from the same target region t1, at different times. As a result, ultrasonic signals output from the respective ultrasonic elements 111 to 115 may have predetermined time differences, and the time-difference corrector 221 may correct the time differences between the ultrasonic signals output from the respective ultrasonic elements 111 to 115. The time-difference corrector 221 may delay transmission of an ultrasonic signal that is input to a predetermined channel, according to predetermined criteria to correct a time difference of ultrasonic waves that are input to the predetermined channel. As shown in FIG. 15, the time-difference corrector 221 may correct time differences of ultrasonic waves that are input to respective channels, through a plurality of delay units D1, D2, D3, D4, and D5. Accordingly, a plurality of ultrasonic signals generated at the same time from the same target region t1 may be transferred at the same time to the ultrasonic signal obtainer 222 or to the focuser 223 of the beamformer 220.

According to an exemplary embodiment, as shown in FIG. 15, the ultrasonic signal obtainer 222 may generate predetermined ultrasonic signals based on the ultrasonic signals transferred from the ultrasonic elements 111 to 115.

The ultrasonic signal obtainer 222 may receive ultrasonic signals of a plurality of channels, whose time differences have been corrected by the time-difference corrector 221, and acquire ultrasonic signals for the plurality of target regions t1 to t3 based on the ultrasonic signals of the plurality of channels. According to an exemplary embodiment, the ultrasonic signal obtainer 222 may acquire ultrasonic signals for the plurality of target regions t1 to t3, based on ultrasonic waves transmitted to the plurality of target regions t1 to t3 and ultrasonic waves received from the plurality of target regions t1 to t3. The ultrasonic signal obtainer 222 may acquire ultrasonic signals for the respective target regions t1 to t3 spaced by a predetermined distance. For example, as shown in FIG. 7A-7C, the ultrasonic signal obtainer 222 may acquire ultrasonic signals for the respective target regions t1 to t3 based on the acquired ultrasonic signals and the transmitted ultrasonic waves. Also, as shown in FIGS. 7A to 12, the ultrasonic signal obtainer 222 may first acquire ultrasonic signals for predetermined target regions, for example, the odd-numbered target regions t1 and t3 among the plurality of target regions t1 to t3, output or store the ultrasonic signals, successively acquire an ultrasonic signal for the other target region, for example, the even-numbered target region t2, and then output or store the ultrasonic signal, thereby acquiring ultrasonic signals for all of the target regions t1 to t3. The acquired ultrasonic signals may be transferred to the focuser 223 as shown in FIG. 15.

As shown in FIG. 15, the focuser 223 may receive ultrasonic signals of a plurality of channels from the ultrasonic signal obtainer 222, and focus the ultrasonic signals of the plurality of channels. The focuser 223 may focus the ultrasonic signals after allocating a predetermined weight (for example, a beamforming coefficient) to an ultrasonic signal of each channel to enhance signals of predetermined locations or relatively attenuate signals of other locations. Accordingly, an ultrasonic image that meets user's requirements or convenience can be generated. The focuser 223 may focus ultrasonic signals using predetermined beamforming coefficients, regardless of the ultrasonic signals generated by the ultrasonic signal obtainer 222. The focuser 223 may calculate optimal beamforming coefficients based on ultrasonic signals generated by the ultrasonic signal obtainer 222, and focus ultrasonic signals using the optimal beamforming coefficients. The focuser 223 may transfer the focused ultrasonic signal z to the image processor 230.

According to an exemplary embodiment, as shown in FIG. 16, the focuser 223 may receive ultrasonic signals of a plurality of channels output from the ultrasonic elements 111 to 115 and subjected to time-difference correction by the time-difference corrector 221, focus the ultrasonic signals of the plurality of channels, and outputs the focused ultrasonic signal. For example, the focuser 223 may focus the ultrasonic signals using predetermined beamforming coefficients regardless of ultrasonic signals output from the ultrasonic elements 111 to 115. Alternatively, the focuser 223 may calculate optimal beamforming coefficients using ultrasonic signals output from the ultrasonic elements 111 to 115, and focus ultrasonic signals using the optimal beamforming coefficients. The ultrasonic signals focused by the focuser 223 may be transferred to the ultrasonic signal obtainer 222, as shown in FIG. 16.

For example, the ultrasonic signal obtainer 222 may receive the focused ultrasonic signal, and acquire ultrasonic signals for the plurality of target regions t1 to t3 based on the focused ultrasonic signal and ultrasonic waves transmitted to the plurality of target regions t1 to t3. In the same way as described above, the ultrasonic signal obtainer 222 may acquire ultrasonic signals for the respective target regions t1 to t3 spaced by a predetermined distance, or the ultrasonic signal obtainer 222 may first acquire ultrasonic signals for predetermined target regions of the plurality of target regions t1 to t3, and successively acquire ultrasonic signals of the other target regions to thereby acquire ultrasonic signals for all of the target regions t1 to t3. The ultrasonic signal obtainer 222 may transfer the acquired ultrasonic signals to the image processor 230.

The ultrasonic signals output from the ultrasonic signal obtainer 222 or the focuser 223 of the beamformer 220 may be transferred to the image processor 230. According to an exemplary embodiment, the ultrasonic signals output from the ultrasonic signal obtainer 222 or the focuser 223 may be transferred to the storage unit 240.

The image processor 230 may generate an ultrasonic image based on the ultrasonic signals. The image processor 230 may generate ultrasonic images of various modes, such as an amplitude mode (A mode) or a brightness mode (B mode), based on the beamformed ultrasonic signals. An ultrasonic image of an A mode is an ultrasonic image expressed with amplitudes. The ultrasonic image of the A mode may be obtained by representing the intensities of reflection as amplitudes based on a distance between the target region t1 and the ultrasonic probe 100 or a reflection time of ultrasonic waves between the target region t1 and the ultrasonic probe 100. An ultrasonic image of a B mode may be obtained by representing the magnitudes of ultrasonic waves as brightness values. The image processor 230 may combine ultrasonic signals corresponding to ultrasonic waves generated at different target regions in order to generate an ultrasonic image from beamformed ultrasonic signals. The image processor 230 may perform post-processing on the ultrasonic image to correct the ultrasonic image, according to a user's intention or convenience. For example, the image processor 230 may correct the brightness, contrast, and/or color of the entire or a part of an ultrasonic image so that a user can clearly view tissue in the ultrasonic image. The image processor 230 may generate a stereo ultrasonic image using a plurality of ultrasonic images.

The ultrasonic image generated or corrected by the image processor 230 may be transferred to the storage unit 240, as shown in FIG. 14. The storage unit 240 may temporarily or non-temporarily store ultrasonic signals output from the beamformer 220, or ultrasonic images for images generated by or generated and corrected by the image processor 230. The storage unit 240 may store the ultrasonic images as a raw data or as an image data (for example, a plurality of graphical image files or video files) processed through predetermined image processing and conversion.

The input unit 188 may receive a predetermined instruction or command from a user to control the ultrasonic imaging apparatus. The input unit 188 may be installed in the main body 200, or physically separated from the main body 200. For example, the input unit 188 may be installed in a workstation connected to the main body 200 through a wired and/or wireless communication network. If the input unit 188 is physically separated from the main body 200, the input unit 188 may transfer a user's instruction or command received through the wired and/or wireless communication network that enables data transmission and reception to and from the main body 200, to the main body 200.

The input unit 188 may include various user interfaces, such as a keyboard, a mouse, a trackball, a touch screen, or a paddle.

The display 190 may display an ultrasonic image generated or corrected by the image processor 230, or an ultrasonic image stored in the storage unit 240, on a screen. According to an exemplary embodiment, the display 190 may display a stereo ultrasonic image on a screen. The display 190 may be a monitor installed in the ultrasonic imaging apparatus, as shown in FIG. 13, or may be a monitor of a workstation connected to the ultrasonic imaging apparatus through a wired and/or wireless communication network.

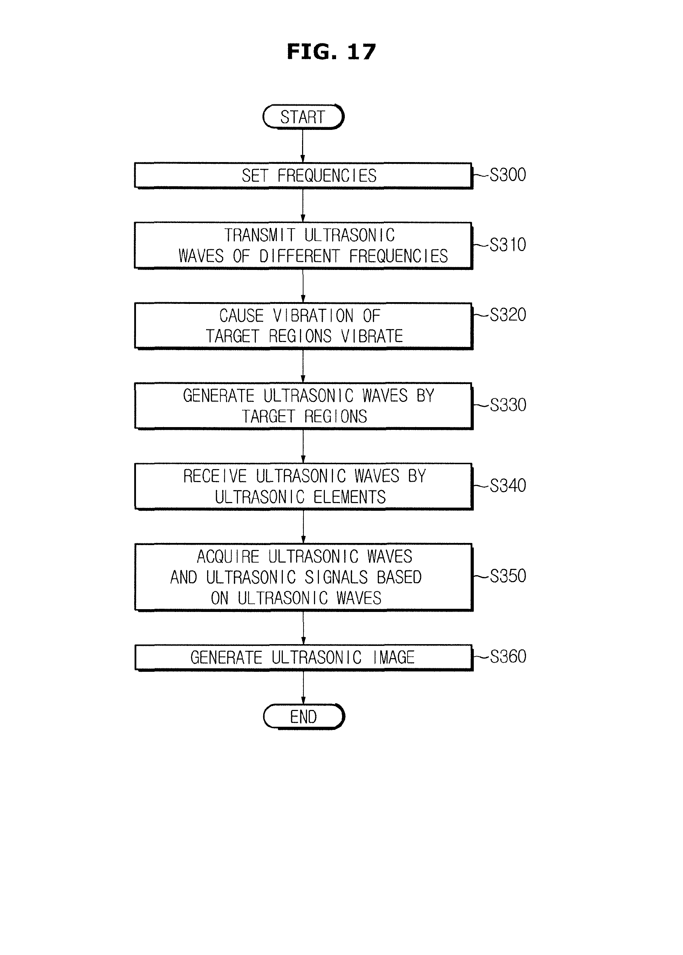

Hereinafter, a control method of the ultrasonic imaging apparatus will be described with reference to FIGS. 17 to 19. FIG. 17 is a flowchart illustrating a control method of the ultrasonic imaging apparatus, according to an exemplary embodiment.

As shown in FIG. 17, in order to control the ultrasonic imaging apparatus, a frequency of ultrasonic waves that are to be transmitted by a plurality of ultrasonic elements may be set. For example, a plurality of different ultrasonic frequencies may be set, in operation S300.

When the different ultrasonic frequencies are set, a plurality of ultrasonic waves of the different frequencies may be transmitted at the same time using a plurality of target regions as focal points, in operation S310.

The plurality of ultrasonic waves of the different frequencies may interfere to generate interference ultrasonic waves. The interference ultrasonic waves may arrive at the plurality of target regions that are focal points, and the plurality of target regions at which the interference ultrasonic waves have been arrived may vibrate in operation 320 to generate ultrasonic waves in operation S330.

The ultrasonic waves may be received by the plurality of ultrasonic elements, and the plurality of ultrasonic elements may convert the received ultrasonic waves into electrical signals of a plurality of channels, that is, ultrasonic signals, and then output the ultrasonic signals, in operation S340.

Successively, the ultrasonic signals for the respective target regions are acquired based on the ultrasonic waves transmitted and the ultrasonic wave received, in operation S350. As described above with reference to FIGS. 7 to 12, the ultrasonic waves transmitted and the ultrasonic wave received may be synthesized to acquire ultrasonic signals for the individual target regions.

An ultrasonic image for the corresponding object may be generated based on the ultrasonic signals, in operation S360.

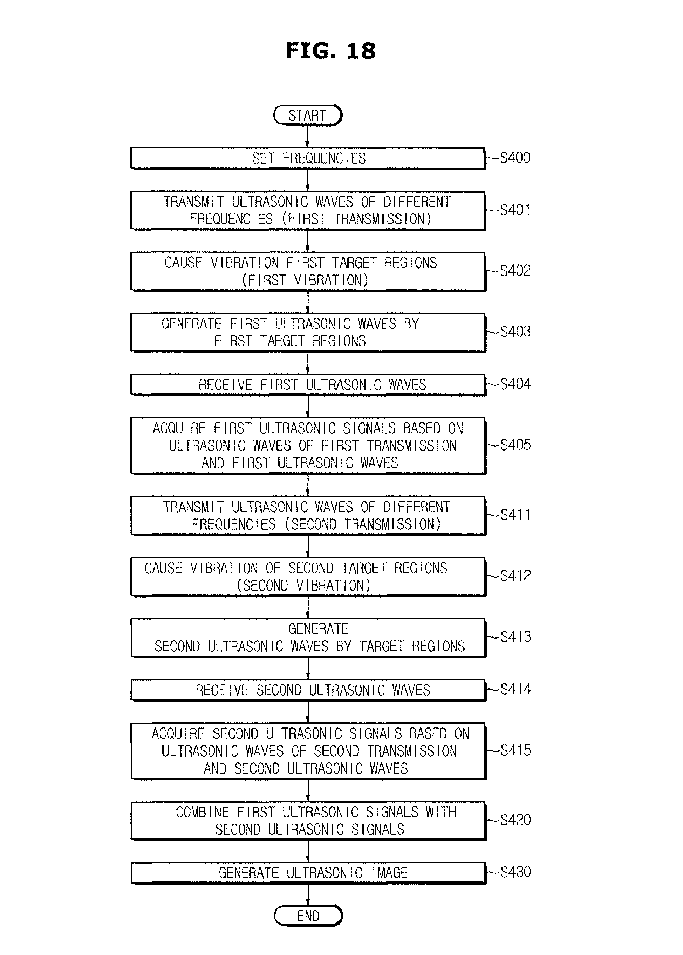

FIG. 18 is a flowchart illustrating a control method of the ultrasonic imaging apparatus, according to an exemplary embodiment.

A plurality of frequencies of ultrasonic waves that are to be transmitted may be set, in operation S400. The frequencies may be different from each other.

A plurality of ultrasonic waves of the plurality of different frequencies may be generated, and the ultrasonic waves of the different frequencies may be focused on and transmitted to a plurality of first target regions (first transmission), in operation S401. The plurality of first target regions may be spaced at predetermined intervals.

The plurality of ultrasonic waves of the different frequencies may interfere to generate interference frequencies, and the plurality of first target regions at which the interference frequencies have been arrived may vibrate (first vibration) at a predetermined frequency according to the frequency of the interference ultrasonic waves in operation S402 to generate first ultrasonic waves in operation S403.

A plurality of ultrasonic elements may receive the first ultrasonic waves, convert the received first ultrasonic waves into electrical signals and output the electrical signals, in operation S404.

Successively, first ultrasonic signals may be acquired based on the ultrasonic waves of the different frequencies used in the first transmission, in operation S405. Like the above-described exemplary embodiment, ultrasonic signals for the first target regions may be acquired based on the plurality of ultrasonic waves and the plurality of ultrasonic signals. According to an exemplary embodiment, the ultrasonic signals used in operation S405 may be beamformed ultrasonic signals.

Then, a plurality of ultrasonic waves of a plurality of different frequencies may be again generated, and the ultrasonic waves of the different frequencies may be focused on and transmitted to a plurality of second target regions (second transmission), in operation S411. For example, according to exemplary embodiments, a plurality of ultrasonic waves of the different frequencies that are the same as in the first transmission may be again generated and transmitted, or a plurality of ultrasonic waves of a plurality of frequencies that are different from the frequencies of the first transmission may be generated and transmitted. The plurality of second target regions on which the ultrasonic waves of the plurality of frequencies are focused upon the second transmission may be different from the plurality of first target regions on which the ultrasonic waves of the plurality of frequencies are focused upon the first transmission. The plurality of second target regions may be spaced by a predetermined distance.

The transmitted ultrasonic waves of the different frequencies may interfere to generate interference ultrasonic waves. The interference ultrasonic waves may arrive at the plurality of second target regions, and the plurality of second target regions may vibrate according to the frequency of the interference ultrasonic waves (second vibration), in operation S412. Due to the vibration of the plurality of second target regions, second ultrasonic waves may be generated from the plurality of second target regions, in operation S413.

The second ultrasonic waves may be received by the ultrasonic elements, in operation S414. The ultrasonic elements may convert the received second ultrasonic waves into electrical signals. According to an exemplary embodiment, the electrical signals may be beamformed.

Successively, second ultrasonic signals may be acquired based on the plurality of ultrasonic waves of the different frequencies transmitted in the second transmission and the converted signals, in operation S415.

The first ultrasonic signals acquired upon the first transmission may be combined with the second ultrasonic signals acquired upon the second transmission to acquire ultrasonic signals for the first target regions and the second target regions, in operation S420.

If the ultrasonic signals for the first target regions and the second target regions are acquired, an ultrasonic image may be generated based on the acquired ultrasonic signals, in operation S430.

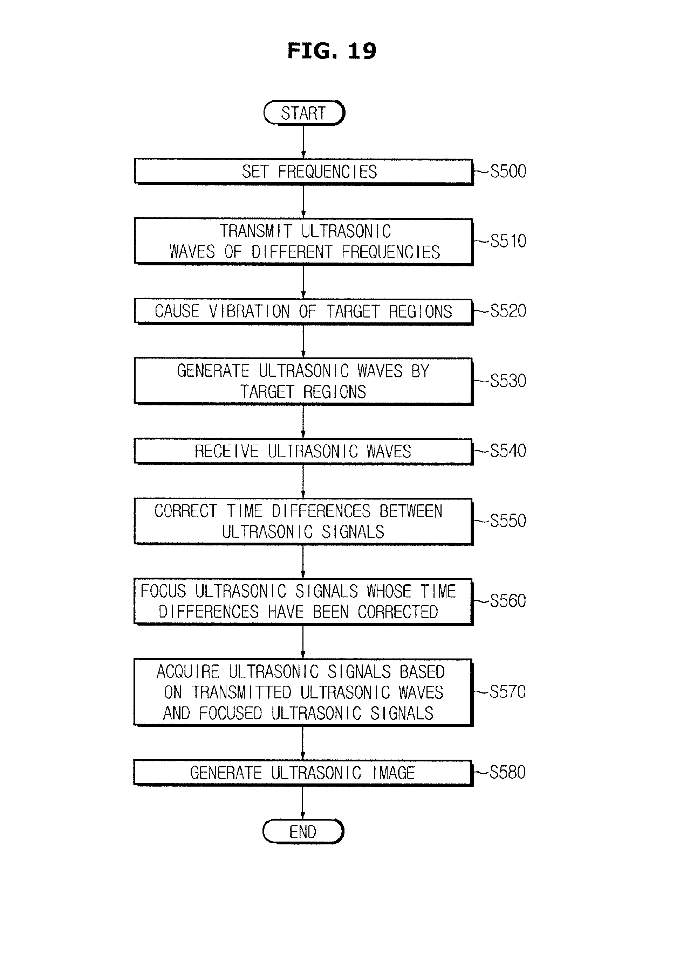

FIG. 19 is a flowchart illustrating a control method of the ultrasonic imaging apparatus, according to an exemplary embodiment.

A plurality of frequencies of ultrasonic waves that are to be transmitted by the ultrasonic elements may be set, in operation S500. The frequencies may be different from each other.

When the plurality of different frequencies are set, a plurality of ultrasonic waves of the plurality of different frequencies may be transmitted using a plurality of target regions as focal points, in operation S510.

If the plurality of ultrasonic waves of the plurality of different frequencies are transmitted, the plurality of ultrasonic waves of the plurality of different frequencies may interfere to generate interference ultrasonic waves. The interference ultrasonic waves may arrive at a plurality of target regions, and the plurality of target regions may vibrate according to the frequency of the interference ultrasonic waves, in operation S520.

Accordingly, ultrasonic waves may be generated from the plurality of target regions, in operation S530.

The ultrasonic waves generated from the plurality of target regions may be received and collected by the ultrasonic elements, etc., in operation S540. The ultrasonic elements may convert the ultrasonic waves into predetermined electrical signals of a plurality of channels, that is, ultrasonic signals of a plurality of channels, and output the ultrasonic signals of the plurality of channels.

After the ultrasonic signals of the plurality of channels are output, time differences between the ultrasonic signals of the channels may be corrected, in operation S550. The ultrasonic signals whose time differences have been corrected may be focused to acquire focused ultrasonic signals, in operation S560. In order to focus the ultrasonic signals, predetermined beamforming coefficients may be used.

The ultrasonic signals for the individual target regions may be acquired based on the focused ultrasonic signals and the plurality of ultrasonic waves transmitted to the target regions, in operation S570. As described above with reference to FIGS. 7 to 12, ultrasonic signals for the individual target regions may be acquired based on the focused ultrasonic signals and the plurality of ultrasonic waves transmitted to the target regions.

After the ultrasonic signals for the individual target regions are acquired, an ultrasonic image may be generated based on the acquired ultrasonic signals, in operation S580.

Therefore, according to the ultrasonic probe, the ultrasonic imaging apparatus, and the control method of the ultrasonic imaging apparatus, as described above, it is possible to acquire ultrasonic signals required to generate an ultrasonic image with a small number of times of the ultrasonic wave transmissions.

Also, ultrasonic images can be quickly acquired.

Furthermore, ultrasonic images of high definition can be obtained.

In addition, since ultrasonic waves can be received without using a hydrophone to generate ultrasonic images, the complexity of the ultrasonic imaging apparatus can be reduced, and the manufacturing cost of the ultrasonic imaging apparatus can also be reduced.

The foregoing exemplary embodiments and advantages are merely exemplary and are not limiting. The present teaching can be readily applied to other types of apparatuses. The description of the exemplary embodiments is intended to be illustrative, and not to limit the scope of the claims, and many alternatives, modifications, and variations will be apparent to those skilled in the art.

**Claims**

1. An ultrasonic probe comprising:

   an ultrasonic array including ultrasonic elements configured to receive ultrasonic waves that are generated from target regions according to interference between the ultrasonic waves of different frequencies which are transmitted to same target regions; and
   a support frame on which the ultrasonic elements are arranged.

2. The ultrasonic probe according to claim 1, wherein the ultrasonic elements comprise:

   ultrasonic generating elements configured to transmit the ultrasonic waves of the different frequencies to the target regions; and
   ultrasonic receiving elements configured to receive the ultrasonic waves that are generated from the target regions.

3. The ultrasonic probe according to claim 2, wherein the ultrasonic generating elements transmit the ultrasonic waves of the different frequencies to the target regions spaced by a distance, at a same time; or
   wherein the ultrasonic generating elements transmit the ultrasonic waves of the different frequencies to different target regions, at a same time, several times.

4. The ultrasonic probe according to one of claims 1 - 3, wherein the ultrasonic elements are arranged in a row on the support frame.

5. The ultrasonic probe according to one of claims 1 - 4, further comprising:

   an ultrasonic signal obtainer configured to acquire ultrasonic signals for the target regions based on the transmitted ultrasonic waves of the different frequencies and the received ultrasonic waves, preferably
   wherein the ultrasonic signal obtainer is configured to acquire the ultrasonic signals for the target regions according to the target regions from which the ultrasonic waves are generated; and/or
   wherein the ultrasonic signal obtainer is configured to acquire the ultrasonic signals for the target regions, based on focused ultrasonic signals acquired by focusing ultrasonic signals corresponding to the received ultrasonic waves and the transmitted ultrasonic waves of the different frequencies.

6. The ultrasonic probe according to one of claims 1 - 5, wherein a frequency of the ultrasonic waves generated from the target regions is lower than that of the ultrasonic waves transmitted to the same target regions.

7. The ultrasonic probe according to one of claims 1 - 6, wherein the ultrasonic waves received by the ultrasonic elements include a harmonic component that is generated when the ultrasonic waves generated from the target regions pass through a medium.

8. An ultrasonic imaging apparatus comprising:

   the ultrasonic probe of one of claims 1 - 7, including the ultrasonic elements configured to receive ultrasonic waves that are generated from target regions according to interference between the ultrasonic waves of different frequencies which are transmitted to same target regions; and
   an ultrasonic signal obtainer configured to acquire ultrasonic signals for the target regions, based on the ultrasonic waves of the different frequencies transmitted to the target regions and the received ultrasonic waves.

9. The ultrasonic imaging apparatus according to claim 8, further comprising:

a focuser configured to focus ultrasonic signals corresponding to the received ultrasonic waves or to focus ultrasonic signals for the target regions acquired by the ultrasonic signal obtainer, preferably, wherein the focuser is configured to focus filtered ultrasonic signals acquired by filtering the ultrasonic signals corresponding to the received ultrasonic waves or by filtering the ultrasonic signals for the target regions acquired by the ultrasonic signal obtainer.

10. The ultrasonic imaging apparatus according to one of claims 8 or 9, wherein the ultrasonic signal obtainer is configured to acquire ultrasonic signals for the target regions according to the target regions from which the ultrasonic waves have been generated.

11. The ultrasonic imaging apparatus according to one of claims 8 - 10, wherein a portion of the ultrasonic elements is configured to generate a grating lobe, or different groups of the ultrasonic elements are configured to transmit the ultrasonic waves of different frequencies to the target regions spaced away from each other, at a same time.

12. The ultrasonic imaging apparatus according to one of claims 8 - 11, wherein the ultrasonic elements transmit the ultrasonic waves of the different frequencies to different target regions, several times.

13. The ultrasonic imaging apparatus according to one of claims 8 - 12, wherein the ultrasonic waves received by the ultrasonic elements include a harmonic component that is generated when the ultrasonic waves generated from the target regions pass through a medium.

14. A control method of an ultrasonic imaging apparatus, the control method comprising:

transmitting ultrasonic waves of different frequencies to target regions, at a same time;
receiving ultrasonic waves generated from the target regions according to interference between ultrasonic waves of the different frequencies which are transmitted to same target regions;
acquiring ultrasonic signals for the target regions based on the ultrasonic waves of the different frequencies and the received ultrasonic waves; and
generating an ultrasonic image based on the acquired ultrasonic signals.

15. The control method according to claim 14, wherein the transmitting the ultrasonic waves of the different frequencies comprises:

transmitting the ultrasonic waves of the different frequencies to the target regions spaced by a distance; and/or
wherein the acquiring the ultrasonic signals for the target regions comprises:
acquiring the ultrasonic waves for the target regions according to the target regions from which the ultrasonic waves are reflected.

# FIG. 1

# FIG. 2

EP 2 933 795 A1

# FIG. 3A

## FIG. 3B

# FIG. 4

# FIG. 5

# FIG. 6

# FIG. 7A

d

t1    t2    t3

TRANSMITTED
ULTRASONIC WAVES

# FIG. 7B

t1     t2     t3

RECEIVED
ULTRASONIC WAVES

# FIG. 7C

t1    t2    t3
●      ●      ●

ACQUIRED
ULTRASONIC SIGNAL

# FIG. 8A

d

t1     t2     t3

TRANSMITTED
ULTRASONIC WAVES

# FIG. 8B

t1      t2      t3

RECEIVED
ULTRASONIC WAVES

# FIG. 8C

t1      t2      t3

●      ●      ●

ACQUIRED
ULTRASONIC SIGNAL

# FIG. 9

# FIG. 10A

TRANSMITTED
ULTRASONIC WAVES

# FIG. 10B

t1     t2     t3

RECEIVED
ULTRASONIC WAVES

# FIG. 10C

t1    t2    t3

ACQUIRED
ULTRASONIC SIGNAL

# FIG. 11

# FIG. 12

# FIG. 13

# FIG. 14

98

t1   t2   t3

ULTRASONIC PROBE
110r

ULTRASONIC
RECEIVING ELEMENT

110t1
FIRST
ULTRASONIC
GENERATING
ELEMENT

110t2
SECOND
ULTRASONIC
GENERATING
ELEMENT

~100

ULTRASONIC
SIGNAL

MAIN BODY

~200

220
BEAMFORMER

211
ULTRASONIC
GENERATION
CONTROLLER

212
POWER
SOURCE

230
IMAGE
PROCESSOR

240
STORAGE
UNIT

210
SYSTEM
CONTROLLER

188
INPUT UNIT

190
DISPLAY

ULTRASONIC IMAGE

**FIG. 15**

BEAMFORMER 220

TIME-DIFFERENCE CORRECTOR 221

ULTRASONIC ELEMENT

ULTRASONIC SIGNAL OBTAIMER 222

FOCUSER 223

98 TARGET REGION (t1)

111 — D1
112 — D2
113 — D3
114 — D4
115 — D5

ULTRASONIC SIGNAL

BEAMFORMED ULTRASONIC SIGNAL(Z)

IMAGE PROCESSOR 230

**FIG. 16**

# FIG. 17

START

SET FREQUENCIES —S300

TRANSMIT ULTRASONIC
WAVES OF DIFFERENT FREQUENCIES —S310

CAUSE VIBRATION OF
TARGET REGIONS VIBRATE —S320

GENERATE ULTRASONIC WAVES BY
TARGET REGIONS —S330

RECEIVE ULTRASONIC WAVES BY
ULTRASONIC ELEMENTS —S340

ACQUIRE ULTRASONIC WAVES
AND ULTRASONIC SIGNALS BASED
ON ULTRASONIC WAVES —S350

GENERATE ULTRASONIC IMAGE —S360

END

# FIG. 18

START

SET FREQUENCIES ~S400

TRANSMIT ULTRASONIC WAVES OF DIFFERENT FREQUENCIES (FIRST TRANSMISSION) ~S401

CAUSE VIBRATION FIRST TARGET REGIONS (FIRST VIBRATION) ~S402

GENERATE FIRST ULTRASONIC WAVES BY FIRST TARGET REGIONS ~S403

RECEIVE FIRST ULTRASONIC WAVES ~S404

ACQUIRE FIRST ULTRASONIC SIGNALS BASED ON ULTRASONIC WAVES OF FIRST TRANSMISSION AND FIRST ULTRASONIC WAVES ~S405

TRANSMIT ULTRASONIC WAVES OF DIFFERENT FREQUENCIES (SECOND TRANSMISSION) ~S411

CAUSE VIBRATION OF SECOND TARGET REGIONS (SECOND VIBRATION) ~S412

GENERATE SECOND ULTRASONIC WAVES BY TARGET REGIONS ~S413

RECEIVE SECOND ULTRASONIC WAVES ~S414

ACQUIRE SECOND ULTRASONIC SIGNALS BASED ON ULTRASONIC WAVES OF SECOND TRANSMISSION AND SECOND ULTRASONIC WAVES ~S415

COMBINE FIRST ULTRASONIC SIGNALS WITH SECOND ULTRASONIC SIGNALS ~S420

GENERATE ULTRASONIC IMAGE ~S430

END

# FIG. 19

START

SET FREQUENCIES — S500

TRANSMIT ULTRASONIC
WAVES OF DIFFERENT FREQUENCIES — S510

CAUSE VIBRATION OF TARGET REGIONS — S520

GENERATE ULTRASONIC WAVES BY
TARGET REGIONS — S530

RECEIVE ULTRASONIC WAVES — S540

CORRECT TIME DIFFERENCES BETWEEN
ULTRASONIC SIGNALS — S550

FOCUS ULTRASONIC SIGNALS WHOSE TIME
DIFFERENCES HAVE BEEN CORRECTED — S560

ACQUIRE ULTRASONIC SIGNALS BASED
ON TRANSMITTED ULTRASONIC WAVES
AND FOCUSED ULTRASONIC SIGNALS — S570

GENERATE ULTRASONIC IMAGE — S580

END

Europäisches
Patentamt

European
Patent Office

Office européen
des brevets

# EUROPEAN SEARCH REPORT

Application Number

EP 15 16 2359

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 779 784 A1 (OLYMPUS CORP [JP]) 2 May 2007 (2007-05-02) | 1,2, 4-11, 13-15 | INV. G10K11/34 |
| Y | * paragraphs [0085] - [0108]; figures 10-15 * | 3,12 | ADD. A61B8/00 |
| X | US 2010/251823 A1 (ADACHI HIDEO [JP] ET AL) 7 October 2010 (2010-10-07) | 1,2, 4-11, 13-15 | |
| Y | * paragraphs [0065], [0066], [0183] - [0185]; figures 4, 20, 22 * | 3,12 | |
| X | WO 99/40847 A2 (TXSONICS LTD [IL]; FRIEDMAN ZVI [IL]; WEINREB ABRAHAM [IL]) 19 August 1999 (1999-08-19) | 1,2, 4-11, 13-15 | |
| Y | * page 10, line 14 - page 13, line 31; figures 1, 2 * | 3,12 | |
| X | US 2011/077524 A1 (OSHIKI MITSUHIRO [JP] ET AL) 31 March 2011 (2011-03-31) | 1,2, 4-11, 13-15 | |
| Y | * paragraphs [0037], [0038], [0085], [0086]; figures 1, 8 * | 3,12 | TECHNICAL FIELDS SEARCHED (IPC) G10K A61B |
| Y | US 2004/102700 A1 (ASAFUSA KATSUNORI [JP]) 27 May 2004 (2004-05-27) * paragraphs [0038], [0039]; figure 2 * | 3,12 | |
| A | EP 2 690 462 A2 (SAMSUNG ELECTRONICS CO LTD [KR]) 29 January 2014 (2014-01-29) * paragraph [0117]; figure 14 * | 4,9 | |

The present search report has been drawn up for all claims

| Place of search | Date of completion of the search | Examiner |
|---|---|---|
| The Hague | 11 September 2015 | Zwerger, Markus |

EPO FORM 1503 03.82 (P04C01)

**ANNEX TO THE EUROPEAN SEARCH REPORT
ON EUROPEAN PATENT APPLICATION NO.**

EP 15 16 2359

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2015

| Patent document cited in search report | | Publication date | Patent family member(s) | | Publication date |
|---|---|---|---|---|---|
| EP 1779784 | A1 | 02-05-2007 | EP | 1779784 A1 | 02-05-2007 |
| | | | JP | 5275565 B2 | 28-08-2013 |
| | | | US | 2007164631 A1 | 19-07-2007 |
| | | | WO | 2005120355 A1 | 22-12-2005 |
| US 2010251823 | A1 | 07-10-2010 | CN | 102197660 A | 21-09-2011 |
| | | | EP | 2346269 A1 | 20-07-2011 |
| | | | JP | 4625145 B2 | 02-02-2011 |
| | | | US | 2010251823 A1 | 07-10-2010 |
| | | | WO | 2010053032 A1 | 14-05-2010 |
| WO 9940847 | A2 | 19-08-1999 | NONE | | |
| US 2011077524 | A1 | 31-03-2011 | JP | 5307453 B2 | 02-10-2013 |
| | | | JP | 2009285125 A | 10-12-2009 |
| | | | US | 2011077524 A1 | 31-03-2011 |
| | | | WO | 2009145087 A2 | 03-12-2009 |
| US 2004102700 | A1 | 27-05-2004 | JP | 4812048 B2 | 09-11-2011 |
| | | | US | 2004102700 A1 | 27-05-2004 |
| | | | WO | 0185031 A1 | 15-11-2001 |
| EP 2690462 | A2 | 29-01-2014 | CN | 103565473 A | 12-02-2014 |
| | | | EP | 2690462 A2 | 29-01-2014 |
| | | | JP | 2014023934 A | 06-02-2014 |
| | | | US | 2014031689 A1 | 30-01-2014 |

EPO FORM P0459

For more details about this annex : see Official Journal of the European Patent Office, No. 12/82